# EUROPEAN PATENT SPECIFICATION

(11) **EP 2 191 895 B1**
(45) Date of publication and mention of the grant of the patent: **24.10.2012**
(21) Application number: 09014415.5
(22) Date of filing: 18.11.2009
(51) Int. Cl.: B03C 1/033, B01L 3/00, G01N 33/543, B03C 1/02, G01N 21/05, B03C 1/28, G01N 15/10

(54) **Microparticle analysis device, microfluidic chip for microparticle analysis, and microparticle analysis method**
Analysegerät für Mikropartikel, mikrofluidischer Chip zur Mikropartikelanalyse und Mikropartikelanalyseverfahren
Dispositif d'analyse de microparticules, puce microfluidique pour analyse de microparticules, et procédé d'analyse de microparticules

(30) Priority: 19.11.2008 JP 2008295086; 07.01.2009 JP 2009001471
(43) Date of publication of application: 02.06.2010
(73) Proprietor: Sony Corporation, Tokyo 108-0075 (JP)
(72) Inventor: Shinoda, Masataka, Tokyo, 108-0075 (JP)
(74) Representative: Müller - Hoffmann & Partner

(56) References cited:
- EP-A- 1 420 251
- EP-A- 1 481 723
- WO-A-98/43066
- WO-A-99/60397
- WO-A-02/084276
- WO-A-03/062787
- WO-A-2008/037995
- WO-A-2008/092859
- WO-A-2008/127292
- KERSAUDY-KERHOAS M ET AL: "Recent advances in microparticle continuous separation" 20080306, vol. 2, no. 1, 6 March 2008 (2008-03-06), pages 1-13, XP006030386

## Description

### BACKGROUND OF THE INVENTION

### 1. Field of the Invention

The present invention relates to micro-fluidic chips for microparticle analysis and microparticle analysis methods.

### 2. Description of Related Art

In recent years, development is being advanced on micro-fluidic chips obtained by providing region and microfluidic channel for performing chemical and biological analysis on a substrate made of silicon or glass by applying microfabrication techniques in the semiconductor industry. These micro-fluidic chips have started to be used as e.g. electrochemical detectors for liquid chromatography and small electrochemical sensors in medical scenes.

The analysis system with such a micro-fluidic chip is referred to as a micro-total-analysis system (µ-TAS), a lab-on-chip, a biochip, and so on, and attracts attention as a technique that allows enhancement in the speed, efficiency, and integration degree of chemical and biological analysis and size reduction of analysis devices.

The µ-TAS has features that analysis can be performed with a small amount of sample and disposable (throwaway) micro-fluidic chips can be used. Therefore, the µ-TAS is expected to be applied to biological analysis in which a tiny amount of precious sample or a large number of specimens is treated particularly.

Application examples of the µ-TAS include a microparticle analysis technique in which characteristics of microparticles such as cells or microbeads are analyzed optically, electrically, or magnetically in a microfluidic channel provided on a micro-fluidic chip. In this microparticle analysis technique, fractional collection of a population (group) satisfying a predetermined condition from microparticles as a result of the analysis is also carried out.

For example, Japanese Patent Laid-open No. 2003-107099 (hereinafter referred to as Patent Document 1) discloses "a microparticle fractionation micro-fluidic chip having a microfluidic channel for introducing a solution containing microparticles and a sheath flow forming microfluidic channel disposed on at least one side of the microfluidic channel." This microparticle fractionation micro-fluidic chip further has "a microparticle measurement part for measuring introduced microparticles, at least two microparticle fractionation microfluidic channels that are disposed on the downstream side of the microparticle measurement part and serve for fractional collection of microparticles, and at least two electrodes that are disposed near a microfluidic channel port from the microparticle measurement part to the microparticle fractionation microfluidic channel and serve to control the movement direction of microparticles."

WO 2008/ 127292 A2 describes screening in a microfluidic device that is mediated by a magnetic field that in some manner displaces or otherwise activates the entities of interest. Entities of interest can be identified and/or separated from one or more other components provided to the microfluidic device. Microfluidic devices may have mechanisms that apply a defined magnetic field to a region of the microfluidic device where library members pass through sequentially and/or in parallel.

### SUMMARY OF THE INVENTION

According to Patent Document 1, the microparticle fractionation micro-fluidic chip and the microparticle fractionation device having this micro-fluidic chip disclosed therein allow fractional collection of a population (group) satisfying a predetermined condition from microparticles. However, analysis by use of this microparticle fractionation device and so on takes a long time because the device is based on a system in which all of the introduced microparticles are measured by the microparticle measurement part and the microparticles satisfying the predetermined condition among the measured microparticles are sorted one by one into the microparticle fractionation microfluidic channel by the electrodes. Therefore, the analysis efficiency is significantly low particularly in the case of sorting a very small number of microparticles satisfying the predetermined condition among a large number of microparticles.

There is a need for the present invention to provide a microparticle analysis device capable of efficiently sorting a population (group) satisfying a predetermined condition from microparticles.

The above-identified problem is solved by a micro-fluidic chip according to claim 1, by the microparticle analysis device according to claim 5 comprising the micro-fluidic chip according to claim 1, and by the method according to claim 7. Further embodiments of the devices are defined in the dependent claims.

According to the present invention, there is provided a micro-fluidic chip according to claim 1.

This microparticle analysis chip allows the following procedure. Specifically, the sample liquid containing the magnetic microparticle and the non-magnetic microparticle is introduced into the region on the micro-fluidic chip, having the internal space in which a magnetic field is formed by the magnetic field generator. Furthermore, the magnetic microparticle that does not pass through a magnetic space generated by the magnetic field generator is held in the region and simultaneously the non-magnetic microparticle that passes through the magnetic space generated by the magnetic field generator is sent to the microfluidic channel connected to the region on the downstream side in the liquid sending direction. In addition, a characteristic of the microparticle may be analyzed by an optical, electrical, or magnetic detector in the microfluidic channel.

Furthermore, a microparticle analysis device allows the following procedure. Specifically, the sample liquid containing the magnetic microparticle and the non-magnetic microparticle is introduced into the region on the micro-fluidic chip, having the internal space in which a magnetic field is formed by the magnetic field generator. Furthermore, the magnetic microparticle that does not pass through a magnetic space generated by the magnetic field generator is held in the region and simultaneously the non-magnetic microparticle that passes through the magnetic space generated by the magnetic field generator is sent to the microfluidic channel connected to the region on the downstream side in the liquid sending direction. In addition, the non-magnetic microparticle is collected.

Moreover, a microparticle analysis device allows the following procedure. Specifically, the sample liquid containing the magnetic microparticle and the non-magnetic microparticle is introduced into the region on the micro-fluidic chip, having the internal space in which a magnetic field is formed by the magnetic field generator. Furthermore, the magnetic microparticle that does not pass through a magnetic space generated by the magnetic field generator is held in the region and simultaneously the non-magnetic microparticle that passes through the magnetic space generated by the magnetic field generator is sent to the microfluidic channel connected to the region on the downstream side in the liquid sending direction. Thereafter, the magnetic field formation by the magnetic field generator is stopped to send the magnetic microparticle held in the region to the microfluidic channel, and the magnetic microparticle is collected.

In this microparticle analysis device, the micro-fluidic chip of (1) includes a microtube that is connected to the region and introduces a sample liquid sent from the region into the microfluidic channel to form a sample liquid laminar flow in a sheath liquid laminar flow passing through the microfluidic channel. Due to this feature, the liquid can be sent to the microfluidic channel in such a way that the sample liquid laminar flow introduced from the microtube is surrounded by the sheath liquid laminar flow.

The microfluidic channel of the micro-fluidic chip of
(1) has a narrowing part at a position that is closer to the downstream side in the liquid sending direction than the position of introduction of a sample liquid by the microtube and is closer to the upstream side in the liquid sending direction than a detection part at which detection of a characteristic of a microparticle by the detector is carried out, and the microfluidic channel inner diameter of the narrowing part is decreased gradually or in a stepwise manner in the liquid sending direction. Due to this feature, the sheath liquid laminar flow and the sample liquid laminar flow can be sent to the detection part in such a way that the laminar flow widths thereof are narrowed. Due to the narrowed sample liquid laminar flow, the liquid can be sent in such a way that the microparticles contained in the sample liquid are continuous with each other on one line in the microfluidic channel. Thus, at the detection part, at which detection of a characteristic of the microparticle by the detector is carried out, the microparticles can be individually analyzed optically, electrically, or magnetically one by one.

The microfluidic channel of the micro-fluidic chip
(1) has two sheath liquid discharge paths arising from branching into trifurcate paths from the microfluidic channel as the center of the branching at a branch part closer to the downstream side in the liquid sending direction than the detection part. This feature allows a part of the sheath liquid laminar flow to be separated from the microfluidic channel after passing through the detection part. These sheath liquid discharge paths can separate the sheath liquid introduced into the microfluidic channel with a large amount from the sample liquid laminar flow. Thus, dilution of the sample liquid by the excess sheath liquid can be prevented, and the concentration of the collected sample liquid can be kept at proper concentration.

A microparticle analysis device may include a liquid sending controller configured to return a sample liquid sent to a position closer to the downstream side in the liquid sending direction than the branch part of the microfluidic channel into the region or introduce the sample liquid into a collection tank. Due to the return of the sample liquid, the microparticle in the sample liquid passes through the magnetic space generated by the magnetic field generator in the region again. This can enhance the purity of the separation between the magnetic microparticle and the non-magnetic microparticle in the sample liquid.

In the micro-fluidic chip of (1), the space inner diameter of the internal space of the region along the direction of a magnetic field formed by the magnetic field generator may be increased gradually or in a stepwise manner in the liquid sending direction. Due to this feature, magnetic acting force between the magnetic microparticle contained in the sample liquid introduced into the region and the magnetic field is increased gradually or in a stepwise manner in the liquid sending direction. This feature can prevent excess deposition of the magnetic microparticle on the sample liquid introduction part in the internal space of the region, and thus can evenly hold the magnetic microparticle in the internal space of the region.

In the micro-fluidic chip of (1), the region can be formed as a turn-back microfluidic channel made in a magnetic space generated by the magnetic field generator. Due to the turn-back microfluidic channel, the microparticle in the sample liquid passes through the magnetic space generated by the magnetic field generator in the region again. This can enhance the purity of the separation between the magnetic microparticle and the non-magnetic microparticle in the sample liquid.

In the micro-fluidic chip of (1), small projections and recesses may be processed on a region surface facing the internal space of the region or a film composed of a magnetic material may be formed on the region surface. Due to the region surface on which the small projections and recesses are processed, the effective area of the magnetic field generator can be increased, and the magnetic microparticle can be evenly held in the internal space of the region. Furthermore, by forming the film composed of a magnetic material on the region surface facing the internal space of the region, the efficiency of the magnetic field generator can be enhanced, and the magnetic microparticle can be efficiently held. According to the invention, in the micro-fluidic chip of (1), a magnetic tube having a hollow shape can be provided in the internal space of the region. By providing the magnetic tube in such a way that its longitudinal direction corresponds with the liquid sending direction, the flow of the microparticle in the internal space can be adjusted. In addition, the magnetic microparticle can be efficiently held on the hollow surface and outside surface of the magnetic tube.

According to other embodiments of the present invention, there are provided the micro-fluidic chip of claim 1 and a microparticle analysis device including this micro-fluidic chip, (2) magnetic field generating means for forming a magnetic field in the internal space of the region of the micro-fluidic chip, and (3) detecting means for detecting an optical, electrical, or magnetic characteristic of a microparticle passing through the microfluidic channel.

The embodiments of the present invention provide a microparticle analysis device capable of efficiently sorting a population (group) satisfying a predetermined condition from microparticles and analyzing only the sorted microparticles optically, electrically, or magnetically.

### BRIEF DESCRIPTION OF THE DRAWINGS

FIG. 1 is a schematic diagram for explaining the configuration of a microparticle analysis device according to an embodiment of the present invention;
FIG. 2 is a schematic diagram for explaining the configuration of a micro-fluidic chip for microparticle analysis according to the embodiment of the present invention;
FIG. 3 is a schematic sectional view corresponding to the section along line P-P in FIG. 2;
FIG. 4 is a schematic diagram showing another preferred form of the micro-fluidic chip for microparticle analysis according to the embodiment of the present invention, in which hollow magnetic tubes are provided in the internal space of a region;
FIGS. 5A to 5D are schematic diagrams for explaining preferred structures of a magnetic tube, FIGS. 5A to 5C are perspective views and FIG. 5D is a sectional view;
FIGS. 6A and 6B are schematic sectional views showing a sheath liquid laminar flow and a sample liquid laminar flow formed in a microfluidic channel of a micro-fluidic chip, FIG. 6A corresponds to the section along line Q-Q in the enlarged view of FIG. 2, and FIG. 6B corresponds to the section along line R-R in the enlarged view of FIG. 2;
FIG. 7 is a schematic sectional view that shows plural microtubes packed into a bundle, and corresponds to the section along line R-R in the enlarged view of FIG. 2;
FIGS. 8A and 8B are schematic sectional views showing the sheath liquid laminar flow and the sample liquid laminar flow around the upstream side and the downstream side, respectively, of a narrowing part of the micro-fluidic chip, FIG. 8A corresponds to the section along line M₁-M₁ in FIG. 6A, and FIG. 8B corresponds to the section along line M₂-M₂ in FIG. 6A;
FIGS. 9A and 9B are schematic sectional views showing the narrowing part whose microfluidic channel inner diameter is decreased in a stepwise manner in the liquid sending direction;
FIGS. 10A to 10C are schematic diagrams for explaining a structural example of a branch part for sheath liquid discharge paths, FIG. 10A is a schematic perspective view, FIG. 10B is a sectional view corresponding to the section along line N₁-N₁ in FIG. 10A, and FIG. 10C is a sectional view corresponding to the section along line N₂-N₂ in FIG. 10A;
FIGS. 11A to 11C are schematic diagrams for explaining a structural example of the branch part for the sheath liquid discharge paths, FIG. 11A is a schematic perspective view, FIG. 11B is a sectional view corresponding to the section along line N₁-N₁ in FIG. 11A, and FIG. 11C is a sectional view corresponding to the section along line N₂-N₂ in FIG. 11A;
FIG. 12 is a schematic diagram for explaining a structural example of the branch parts for the sheath liquid discharge paths 26 and 27;
FIG. 13 is a flowchart for explaining the steps of a microparticle analysis method (negative selection) according to the embodiment of the present invention;
FIG. 14 is a flowchart for explaining the steps of the microparticle analysis method (positive selection) according to the embodiment of the present invention; and
FIGS. 15A and 15B are schematic perspective views showing the structures of the micro-fluidic chip a in which the surface of a detection part is recessed.

### DETAILED DESCRIPTION OF THE PREFERRED EMBODIMENT

A preferred embodiment of the present invention will be described below with reference to the drawings. It should be noted that the embodiment to be described below is merely one example of representative embodiments of the present invention and the scope of the present invention is not narrowly interpreted due to this embodiment. The description will be made in the following order.

### 1. Outline of Configuration of Microparticle Analysis Device and Analysis-subject Microparticle

(1) Outline of Device Configuration
(2) Analysis-subject Microparticle

### 2. Details of Configuration and Operation of Microparticle Analysis Device

(1) Separation of Microparticle by Magnetic Field Generator
(2) Formation of Laminar Flow by Microtube
(3) Narrowing of Laminar Flow Width by Narrowing Part
(4) Characteristic Detection by Detector
(5) Concentration of Sample Liquid by Sheath liquid discharge path
(6) Collection or Return of Sample Liquid by Liquid Sending Controller

### 3. Microparticle Analysis Method

(1) Sorting of Magnetically-unlabeled cell (Negative Selection)
   (1-1) Cell Labeling
   (1-2) Cell Separation and Characteristic Detection
   (1-3) Characteristic Determination and Collection or Return
      (1-3-1) Characteristic Determination
      (1-3-2) Collection
      (1-3-3) Return
(2) Sorting of Magnetically-labeled cell (Positive Selection) (2-1) Cell Labeling
   (2-2) Cell Separation and Characteristic Detection
   (2-3) Characteristic Determination and Collection or Return
      (2-3-1) Characteristic Determination
      (2-3-2) Collection
      (2-3-3) Return

### 4. Micro-fluidic Chip

### 1. Outline of Configuration of Microparticle Analysis Device and Analysis-subject Microparticle

### (1) Outline of Device Configuration

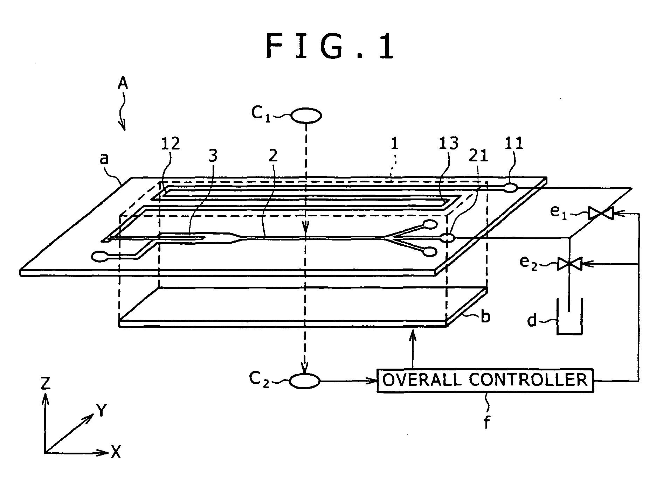

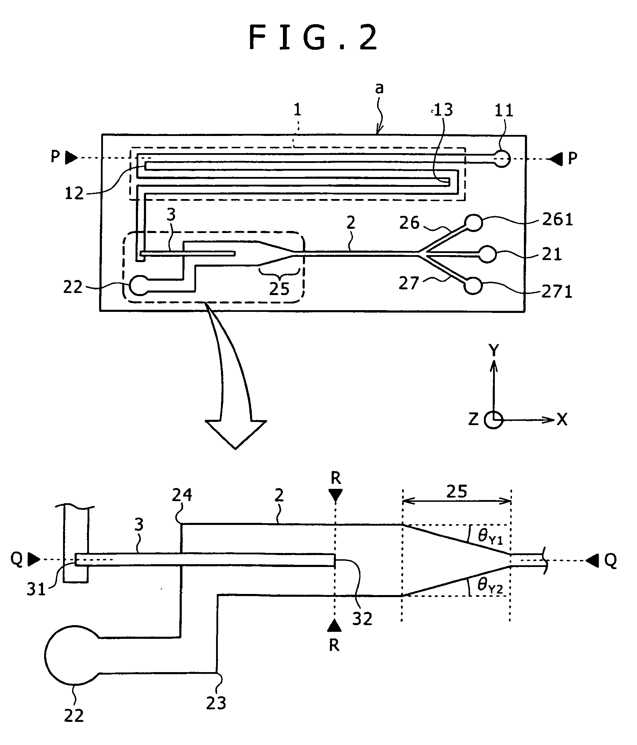

With reference to FIGS. 1 and 2, the outline of the device configuration of a microparticle analysis device according to the embodiment of the present invention will be described below. FIG. 1 is a schematic diagram showing a part of the configuration of the microparticle analysis device according to the present embodiment. FIG. 2 is a schematic diagram showing the configuration of a micro-fluidic chip for microparticle analysis, incorporated in this microparticle analysis device.

Referring to FIG. 1, the microparticle analysis device indicated by symbol A includes a micro-fluidic chip a for microparticle analysis (hereinafter, referred to simply as "micro-fluidic chip a"), a magnetic field generator b that forms a magnetic field in a predetermined region (region 1) of this micro-fluidic chip a, and detectors c₁ and c₂ that detect an optical characteristic of microparticles at a predetermined part (microfluidic channel 2) of the micro-fluidic chip a. Although the magnetic field generator b is so shown in FIG. 1 as to be separated from the micro-fluidic chip a for convenience, it is provided in contact with or be in proximity to the magnetic field formation region of the micro-fluidic chip a.
Specifically, if the magnetic field generator b is provided as an external component of the micro-fluidic chip a, it is disposed at a part in contact with or in proximity to the magnetic field formation region on the device main body. If the magnetic field generator b is provided as an internal component of the micro-fluidic chip a, it is so provided as to be fabricated in the vicinity of the magnetic field formation region on the micro-fluidic chip a.

The micro-fluidic chip a has the region 1 (the region surrounded by the dotted line in FIGS. 1 and 2) into which a sample liquid containing magnetic microparticles and non-magnetic microparticles is introduced. A magnetic field can be formed in the internal space of this region 1 by the magnetic field generator b. In the present embodiment, the region 1 is formed as a turn-back microfluidic channel formed in the magnetic space generated by the magnetic field generator b. In FIGS. 1 and 2, numerals 12 and 13 each indicate a turn-back part of the turn-back microfluidic channel. The region 1 is formed as the turn-back microfluidic channel that is inverted by 180 degrees at the turn-back parts 12 and 13. The region 1 may be obtained by forming the region surrounded by the dotted line as e.g. one straight-line microfluidic channel having large width, and can be formed with any of various shapes in the magnetic space generated by the magnetic field generator b.

The sample liquid introduced from a sample liquid inlet indicated by numeral 11 in FIGS. 1 and 2 into the region 1 by a liquid sending pump (not shown) is sent in the magnetic space generated by the magnetic field generator b via the turn-back parts 12 and 13 and sent to the microfluidic channel (see numeral 2 in the diagrams) formed in communication with the region 1. The communication between the region 1 and the microfluidic channel 2 is obtained via a microtube 3 to be described later.

The sample liquid sent to the microfluidic channel 2 is subjected to detection of a characteristic of microparticles by the detectors c₁ and c₂. The detectors c₁ and c₂ can be formed as an optical detection system, an electrical detection system, or a magnetic detection system similar to one in a microparticle analysis system with use of a micro-fluidic chip of a related art. Specifically, e.g. the optical detection system includes the detector c₁ composed of a laser light source, a condensing lens for focusing laser light on microparticles passing through the microfluidic channel 2 to irradiate the microparticles with the laser light, and so on, and the detector c₂ that detects light generated from the microparticles due to the laser light irradiation by using a dichroic mirror, a bandpass filter, and so on. The detector c₂ may be e.g. a photo multiplier tube (PMT) or an area imaging element such as a CCD (Charge Coupled Device) or a CMOS (Complementary Metal Oxide Semiconductor) element. The light detected by the detector c₂ is converted to an electrical signal and output to an overall controller f. Hereinafter, unless a particular reference is made, the description will be made based on the assumption that the detectors c₁ and c₂ are formed as the optical detection system and detect an optical characteristic of microparticles.

In FIGS. 1 and 2, numeral 21 denotes a sample liquid outlet for discharging the sample liquid from the microfluidic channel 2 to the outside of the micro-fluidic chip a. After the detection of an optical characteristic of the microparticles by the detectors c₁ and c₂, the sample liquid discharged from the sample liquid outlet 21 is sent to a collection tank d or the sample liquid inlet 11. In FIG. 1, symbols e₁ and e₂ denote liquid sending controllers for sending the sample liquid discharged from the sample liquid outlet 21 to either the collection tank d or the sample liquid inlet 11 selectively. The liquid sending controllers e₁ and e₂ can be formed with a generally-used valve, a liquid sending pump, and so on. When the liquid sending controller (valve) e₁ is closed and the liquid sending controller (valve) e₂ is opened, the sample liquid is introduced into the collection tank d. This collection tank d may be provided in plurality. In this case, the liquid sending controller (valve) e₂ is provided with a function to select any one or more collection tanks d and send the sample liquid into the selected tanks d. When the liquid sending controller (valve) e₁ is opened and the liquid sending controller (valve) e₂ is closed, the sample liquid is introduced into the sample liquid inlet 11 and returned into the region 1.

A signal from the overall controller f is output to the magnetic field generator b and the liquid sending controllers e₁ and e₂ (see FIG. 1). In response to this output, the magnetic field generator b forms a magnetic field or stops the formation of a magnetic field. Furthermore, in response to the output, the liquid sending controllers e₁ and e₂ send the sample liquid discharged from the sample liquid outlet 21 to either the collection tank d or the sample liquid inlet 11.

### (2) Analysis-subject Microparticle

In the embodiment of the present invention, the "microparticle" contained as the analysis subject in the sample liquid broadly encompasses biologically relevant microparticles such as cells, microorganisms, and liposomes, synthetic particles such as latex particles, gel particles, and industrial particles, and so on.

The biologically relevant microparticles encompass chromosomes, liposomes, mitochondrias, organelles, and so on included in various kinds of cells. The cells as the subject encompass animal cells (hemocyte cells and so on) and plant cells. The microorganisms encompass bacteria such as coliforms, viruses such as tobacco mosaic viruses, fungi such as yeasts, and so on. Moreover, the biologically relevant microparticles also encompass biologically relevant polymers such as nucleic acids, proteins, and complexes of these substances. The industrial particles may be composed of e.g. an organic or inorganic polymer material or a metal. The organic polymer material encompasses polystyrene, styrene divinylbenzene, polymethylmethacrylate, and so on. The inorganic polymer material encompasses glass, silica, magnetic materials, and so on. The metal encompasses gold colloids, aluminum, and so on. In general, the shape of these microparticles is a sphere. However, it may be a nonspherical shape, and the size, mass, and so on of the microparticles are also not particularly limited.

The "magnetic microparticle" refers to a magnetically-labeled biologically relevant microparticle or a synthetic particle containing e.g. a ferromagnetic material such as iron, cobalt, or nickel or a ferrite such as magnetite or chromite. The "non-magnetic microparticle" refers to a biologically relevant microparticle that is not magnetically labeled or a synthetic particle that contains neither a ferromagnetic material nor a ferrite.

The magnetic labeling of a biologically relevant microparticle can be carried out by using e.g. a magnetic bead antibody. The magnetic bead antibody refers to a material obtained by chemically coupling e.g. a ferromagnetic material or a ferrite formed as a spherical microbead to an antibody having the specific binding ability to a specific substance (antigen) by using a cross-linking agent. Currently, magnetic bead antibodies for various substances are commercially available. By coupling these magnetic bead antibodies to a substance contained in microparticles (particularly, a substance existing on the surface of the microparticles), the microparticles can be magnetically labeled. Furthermore, it will also be possible that the magnetic labeling of a biologically relevant microparticle is carried out e.g. by stirring powders of a ferromagnetic material, a ferrite, or the like and the biologically relevant microparticle under the existence of a flocculant such as poly aluminum chloride to thereby make a magnetic floc (magnetic aggregate).

### 2. Details of Configuration and Operation of Microparticle Analysis Device

The details of the respective device components in the microparticle analysis device A and the operation thereof will be described below by taking as an example the case of performing analysis and sorting of cells with use of a sample liquid containing cells that are magnetically labeled with a magnetic bead antibody as "magnetic microparticles" and cells that are not magnetically labeled with a magnetic bead antibody as "non-magnetic microparticles." The cells that are magnetically labeled with a magnetic bead antibody will be referred to as "magnetically-labeled cells," and the cells that are not magnetically labeled with a magnetic bead antibody will be referred to as "magnetically-unlabeled cells."

### (1) Separation of Microparticle by Magnetic Field Generator

As described with FIG. 1, the sample liquid introduced from the sample liquid inlet 11 into the region 1 by a liquid sending pump (not shown) is sent in the magnetic space generated by the magnetic field generator b via the turn-back parts 12 and 13.

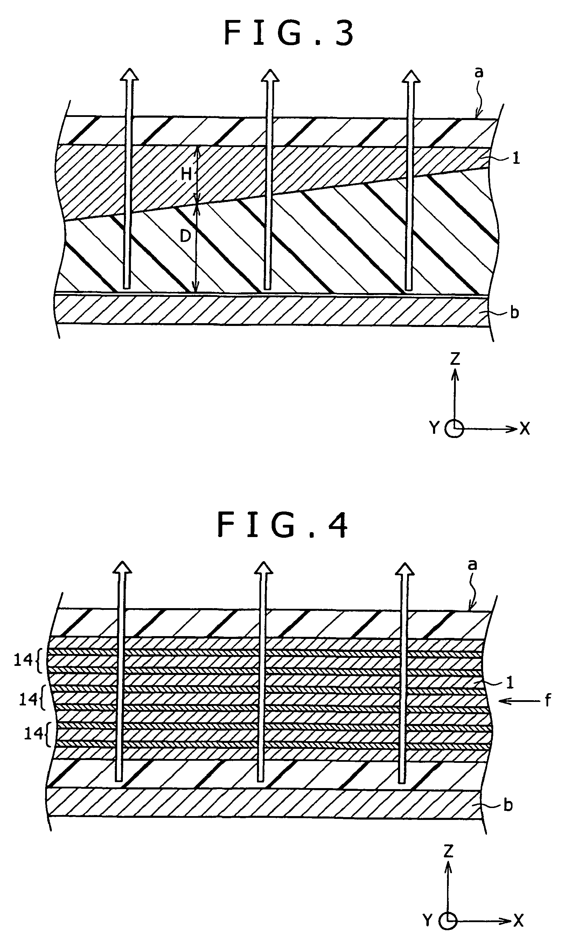

FIG. 3 is a schematic sectional view corresponding to the section along line P-P in FIG. 2. The sample liquid is sent from the right to the left in the diagram (in the X-axis negative direction). The magnetic field generator b is provided in contact with or in proximity to the region 1, and a magnetic field is formed in the internal space of the region 1 by the magnetic field generator b. The block arrowheads in the diagram indicate the direction of the magnetic field formed by the magnetic field generator b.

The magnetic field generator b is formed as a unit capable of controlling formation and stop of a magnetic field, such as an electrically-applied electromagnet. As described above, if the magnetic field generator (electromagnet) b is provided as an external component of the micro-fluidic chip a, it is disposed at a part in contact with or in proximity to the region 1 on the device main body. If the magnetic field generator b is provided as an internal component of the micro-fluidic chip a, it is disposed near the region 1 on the micro-fluidic chip a; for example it is provided below the region 1 as shown in FIG. 3. If the magnetic field generator b is provided as an internal component, it is formed e.g. by fabricating an electromagnet in the substrate of the micro-fluidic chip a or by stacking the electromagnet on the substrate.

If the sample liquid containing the magnetically-labeled cells and the magnetically-unlabeled cells is introduced into the region 1 in the state in which a magnetic field is formed in the internal space of the region 1, magnetic acting force works between the magnetization of the magnetically-labeled cells and the magnetic field. Due to this magnetic acting force, the magnetically-labeled cells are so held in the region 1 as to remain in the internal space of the region 1 against the liquid sending pressure of the sample liquid or be absorbed by the surface facing the internal space of the region 1. On the other hand, the magnetically-unlabeled cells are sent to the microfluidic channel 2 (see FIG. 1), which communicates with the region 1, in accordance with the liquid sending pressure of the sample liquid because the magnetic acting force does not work between the magnetically-unlabeled cells and the magnetic field.

In this manner, the region 1 and the magnetic field generator b exert a function to separate the cells contained in the introduced sample liquid into the magnetically-labeled cells that do not pass through the magnetic space generated by the magnetic field generator b and the magnetically-unlabeled cells that pass through the magnetic space. This makes it possible to separate only the magnetically-unlabeled cells from the cells contained in the sample liquid and send the magnetically-unlabeled cells to the microfluidic channel 2 while a magnetic field is formed by the magnetic field generator b.

If, after the sending of the magnetically-unlabeled cells to the microfluidic channel 2, the magnetic field formed by the magnetic field generator b is stopped to release the magnetic acting force on the magnetically-labeled cells, only the magnetically-labeled cells held in the region 1 can be sent to the microfluidic channel 2.

To surely separate the magnetically-unlabeled cells from the magnetically-labeled cells, it is preferable to hold the magnetically-labeled cells in the region 1 by making them be absorbed by the surface facing the internal space of the region 1 (e.g. the bottom surface of the region 1). For this purpose, the magnetic field generator b forms a magnetic field with such intensity that sufficient magnetic attractive force can be generated between the magnetic field and the magnetization of the magnetically-labeled cells.

If the magnetically-labeled cells are held by making them be absorbed by the surface facing the internal space of the region 1, a larger number of magnetically-labeled cells can be absorbed by the surface by forming small projections and recesses on the surface to thereby increase the surface area. Furthermore, the magnetic attractive force acting on the magnetically-labeled cells can be enhanced by coating the surface facing the internal space of the region 1 with a magnetic material such as iron, cobalt, nickel, or a ferrite by welding, vapor deposition, or another method. Alternatively, by filling the internal space with magnetic beads coated with the magnetic material such as iron, cobalt, nickel, or a ferrite by welding, vapor deposition, or another method, the surface area of the internal space can be increased and thus a larger number of magnetically-labeled cells can be absorbed by the surface.

In the case of holding the magnetically-labeled cells by making them be absorbed by the surface facing the internal space of the region 1, if a large number of magnetically-labeled cells are unevenly absorbed at a certain part of the surface, possibly the internal space of the region 1 is narrowed by the magnetically-labeled cells held at this part and the sending of the sample liquid is inhibited. In particular, a large number of magnetically-labeled cells are contained in the sample liquid at the initial stage of the introduction thereof into the region 1. Therefore, if strong magnetic attractive force acts on the magnetically-labeled cells on the upstream side of the region 1, the magnetically-labeled cells are absorbed by the bottom surface of the region 1 at once and the narrowing easily occurs. To prevent this, it is preferable to allow comparatively-weak magnetic attractive force to act on the magnetically-labeled cells on the upstream side of the region 1 and gradually increase the magnetic attractive force in the flow sending direction toward the downstream side, to thereby permit the magnetically-labeled cells in the sample liquid to be evenly absorbed by the surface.

In the microparticle analysis device A, the space inner diameter of the inside of the region 1 (see symbol H in FIG. 3) along the direction of the magnetic field formed by the magnetic field generator b is gradually increased in the liquid sending direction in order to gradually increase the magnetic attractive force acting on the magnetically-labeled cells. In the case of FIG. 3, in which the magnetic field generator b is disposed below the region 1 and a magnetic field along the Z-axis positive direction is formed in the internal space of the region 1 for making the magnetically-labeled cells be absorbed by the bottom surface of the region 1, the bottom surface of the region 1 is formed as an inclined surface whose surface height in the Z-axis direction is gradually decreased in the liquid sending direction. Due to this feature, the distance between the magnetically-labeled cells passing through the region 1 and the magnetic field generator b (see symbol D in the diagram) is gradually decreased in the liquid sending direction.

When the distance D between the magnetically-labeled cells and the magnetic field generator b is smaller and thus the magnetically-labeled cells are closer to the magnetic field generator b, the magnetic attractive force acting on the magnetically-labeled cells is larger. In contrast, when the distance D between the magnetically-labeled cells and the magnetic field generator b is larger and thus the magnetically-labeled cells are farther away from the magnetic field generator b, the magnetic attractive force acting on the magnetically-labeled cells is smaller. Therefore, if the distance D is gradually decreased in the liquid sending direction, the magnetic attractive force acting on the magnetically-labeled cells passing through the region 1 can be gradually increased and thereby the magnetically-labeled cells can be evenly absorbed by the surface.

In the case of FIG. 3, the magnetic field generator b is disposed below the region 1 and the bottom surface of the region 1 is formed as an inclined surface, to thereby adjust the magnetic attractive force acting on the magnetically-labeled cells. However, the top surface or a side surface facing the internal space of the region 1 may be formed as an inclined surface depending on the position of the magnetic field generator b and the direction of the magnetic field formed by the magnetic field generator b. Furthermore, the surface (bottom surface, top surface, side surface) facing the internal space of the region 1 may be formed as a multi-stage surface having a shape of stairs instead of an inclined surface.

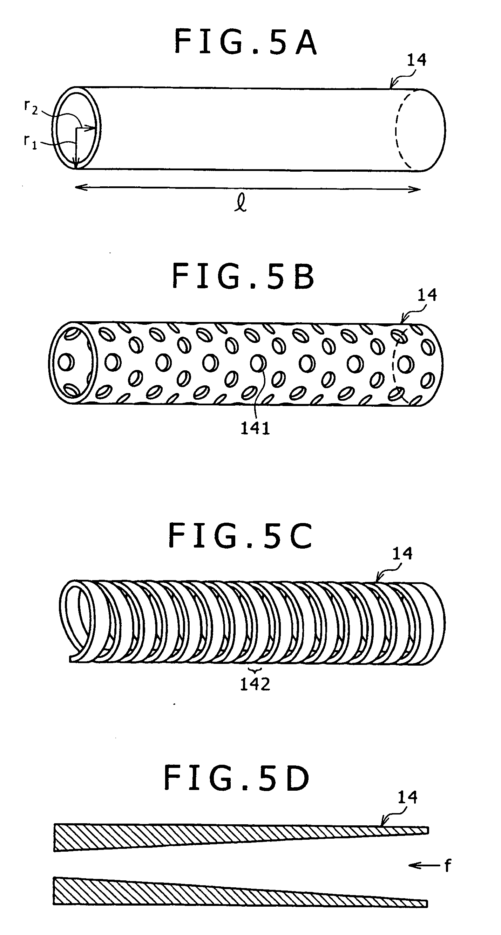

To make the magnetically-labeled cells be absorbed and held in the internal space of the region 1, as shown in FIG. 4, a magnetic tube 14 (see also FIGS. 5A to 5D) having a hollow shape is provided in the internal space of the region 1. The magnetic tube 14 is so provided that its longitudinal direction corresponds with the liquid sending direction (see the arrowhead f in the diagram).

FIG. 4 is a schematic sectional view corresponding to the section along line P-P in FIG. 2A. In the case of FIG. 4, the magnetic tubes 14 each formed into a hollow cylindrical shape are provided in the internal space of the region 1 at three stages in the Z-axis direction. In FIG. 4, the magnetic tubes 14 are provided in plurality also in the Y-axis direction. The number of magnetic tubes 14 provided in the internal space of the region 1 is not particularly limited. The number can be at least one, preferably two or more. The shape of the magnetic tube 14 is not limited to a cylindrical shape as long as it has a hollow shape, and the section thereof may have a polygonal shape such as a triangle or a quadrangle.

The magnetic tube 14 is formed of a magnetic material such as iron, cobalt, nickel, or a ferrite, and is formed into a hollow shape as shown in FIG. 5A. If the magnetic tubes 14 are provided in the internal space of the region 1 as shown in FIG. 4, the magnetically-labeled cells introduced into the region 1 are sent through the hollow of the magnetic tube 14 or the gap between the magnetic tubes 14. In addition, if a magnetic field is formed by the magnetic field generator b, the magnetically-labeled cells sent through the inside and outside of the magnetic tubes 14 are absorbed by the hollow surfaces and outside surfaces of the tubes. If the inner diameter, outer diameter, and length of the magnetic tube 14 are defined as r₁, r₂, and l, respectively, the area S of the surface that can absorb the magnetically-labeled cells, of each magnetic tube 14, is "S = 2nl(r₁ + r₂)." Therefore, similarly to the above-described case in which the internal space of the region 1 is filled with magnetic beads coated with a magnetic material, the area of the surface capable of absorbing the magnetically-labeled cells can be increased and the magnetically-labeled cells can be efficiently held in the internal space of the region 1.

Moreover, the flow of the cells in the internal space of the region 1 can be adjusted by providing the magnetic tubes 14 to thereby allow the cells introduced into the region 1 to be sent through the hollow of the magnetic tube 14 or the gap between the magnetic tubes 14. This feature also makes it possible to prevent inhibition of the sample liquid sending due to the clogging of the cells in the internal space of the region 1.

If small projections and recesses are formed on the hollow surface and outside surface of the magnetic tube 14, the area of the surface capable of absorbing the magnetically-labeled cells can be further increased. Furthermore, it is also possible that, as shown in FIGS. 5B and 5C, small holes 141 are formed in the tube wall of the magnetic tube 14 or the magnetic tube 14 is formed into a coil shape (spring shape) having gaps 142. If such a magnetic tube 14 is provided, the magnetically-labeled cells that move into/from the inside/outside of the magnetic tube 14 via the holes 141 or the gaps 142 can be absorbed by the surfaces of the holes 141 or the gaps 142. This can further increase the area of the surface capable of absorbing the magnetically-labeled cells.

It is preferable that the magnetic tube 14 be formed into a hollow cylinder having small wall thickness in order to suppress the rise of the liquid sending pressure and prevent the clogging of the cells in the tube hollow. Furthermore, it is also possible that, as shown in FIG. 5D, the magnetic tube 14 is formed into a hollow cylinder having a tapered tube wall so that the inner diameter of the tube on the upstream side in the liquid sending direction (see the arrowhead f in the diagram) may be wider whereas the inner diameter on the downstream side be narrower. This is also effective to prevent the clogging of the cells in the tube hollow.

### (2) Formation of Laminar Flow by Microtube

The sample liquid sent in the region 1 is introduced from the region 1 to the microfluidic channel 2 by the microtube (see numeral 3 in FIGS. 1 and 2) connected to the end part of the region 1. The sample liquid introduced to the microfluidic channel 2 contains either the magnetically-unlabeled cells that are separately sent while a magnetic field is formed by the magnetic field generator b or the magnetically-labeled cells that are sent thereafter while the magnetic field formation by the magnetic field generator b is stopped.

In the enlarged view of FIG. 2, numeral 31 denotes an opening of the microtube 3 closer to the end part of the region 1, and numeral 32 denotes an opening of the microtube 3 closer to the microfluidic channel 2. To the microfluidic channel 2, a sheath liquid is introduced from a sheath liquid inlet indicated by numeral 22 in the diagram by a liquid sending pump (not shown). The sheath liquid introduced from the sheath liquid inlet 22 forms a laminar flow and is sent toward the downstream side of the microfluidic channel via bent parts 23 and 24 at which the microfluidic channel is bent by substantially 90 degrees.

The sample liquid supplied from the opening 31 passes through the microtube 3 and is introduced into the sheath liquid laminar flow passing through the microfluidic channel 2 from the opening 32. By introducing the sample liquid into the sheath liquid laminar flow passing through the microfluidic channel 2 by the microtube 3 in this way, the liquids can be sent in such a way that the sample liquid laminar flow is surrounded by the sheath liquid laminar flow.

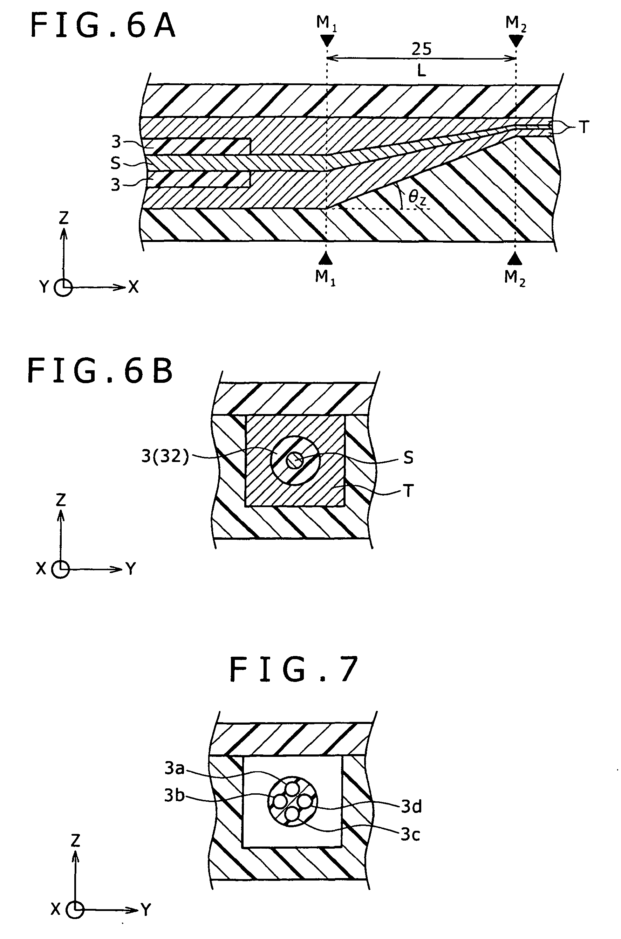

FIGS. 6A and 6B are schematic diagrams showing the sheath liquid laminar flow and the sample liquid laminar flow formed in the microfluidic channel 2. FIG. 6A is a schematic sectional view corresponding to the section along line Q-Q in the enlarged view of FIG. 2, and shows the opening 32 of the microtube 3 and a narrowing part 25 (to be described later) of the microfluidic channel 2 in an enlarged manner. FIG. 6B is a schematic sectional view corresponding to the section along line R-R in the enlarged view of FIG. 2, and shows the opening 32 viewed straightforward from the downstream side of the microfluidic channel 2.

By introducing the sample liquid into the sheath liquid laminar flow (see symbol T in the diagram) passing through the microfluidic channel 2 by the microtube 3, the liquids can be sent in such a way that the sample liquid laminar flow (see symbol S in the diagram) is surrounded by the sheath liquid laminar flow T as shown in FIG. 6A.

In the structure shown in FIGS. 6A and 6B, the microtube 3 is so provided that the center thereof is coaxial with the center of the microfluidic channel 2. In this case, the sample liquid laminar flow S is introduced into the center of the sheath liquid laminar flow T passing through the microfluidic channel 2. The formation position of the sample liquid laminar flow S in the sheath liquid laminar flow T can be set to any position through adjustment of the provision position of the microtube 3 in the microfluidic channel 2.

In the case of FIGS. 6A and 6B, the microtube 3 is provided as one tube. However, the microtube 3 is not limited thereto. For example, as shown in FIG. 7, it may be provided as a bundle of plural tubes (four tubes, in the diagram). If a bundle of tubes is employed as the microtube, for example, the sample liquid can be introduced from any one of microtubes 3a, 3b, 3c, and 3d while solutions other than the sample liquid and the sheath liquid can be introduced from the other microtubes. The number of microtubes packed into a bundle can be two or more and is set to any number depending on the number of solutions to be introduced. At the opening of each microtube on the opposite side to the opening closer to the microfluidic channel 2, an inlet for supplying a solution is provided and a liquid sending pump or the like is connected.

Specifically, e.g. the following usage will be possible. A sample liquid containing magnetically-unlabeled cells or magnetically-labeled cells is introduced from the microtube 3a, and a solution containing a substance that can react with these cells (reactive substance) is introduced from the microtubes 3b, 3c, and 3d, for causing the reaction between the cells and the substance in the laminar flow formed in the microfluidic channel 2. Examples of the reactive substance include an antibody that can be coupled to the cell surface and a compound that can chemically react with the cell.

In the case of detecting an optical characteristic of the cells in the microfluidic channel 2 by the detectors c₁ and c₂, it is also possible to, for example, introduce a fluorescent-labeled antibody that can be coupled to the surfaces of magnetically-unlabeled cells or magnetically-labeled cells from any of the microtubes 3b, 3c, and 3d and cause fluorescent labeling of the cells at the time of laminar flow formation by the microtube 3, to thereby detect the characteristic based on the fluorescent substance. Furthermore, the sample liquid discharged from the sample liquid outlet 21 can be returned to the region 1 by the liquid sending controllers e₁ and e₂. In this case, for example, it is possible that a compound is introduced from the microtube 3b and analysis is performed and thereafter another compound is introduced from the microtube 3c into the returned sample liquid and analysis is performed again.

### (3) Narrowing of Laminar Flow Width by Narrowing Part

The laminar flow widths of the sample liquid and the sheath liquid set to the state in which the sample liquid laminar flow S is surrounded by the sheath liquid laminar flow T by the microtube 3 are narrowed by the narrowing part (see numeral 25 in FIG. 2) formed on the downstream side in the liquid sending direction.

The narrowing part 25 is so formed that the inner diameter of the microfluidic channel gradually decreases in the liquid sending direction from the upstream side of the microfluidic channel toward the downstream side. Specifically, as shown in the enlarged view of FIG. 2, the microfluidic channel in the narrowing part 25 is gradually narrowed in the Y-axis direction along the liquid sending direction, and the narrowing part 25 is formed into a spindle shape in top view. This shape allows the narrowing part 25 to exert the function to narrow the laminar flow widths of the sheath liquid laminar flow T and the sample liquid laminar flow S in the Y-axis direction. Moreover, as shown in FIG. 6A, the narrowing part 25 is so formed that the microfluidic channel bottom surface is an inclined surface whose height in the depth direction (Z-axis direction in the diagram) increases in the liquid sending direction. This can narrow the laminar flow widths also in the Z-axis direction.

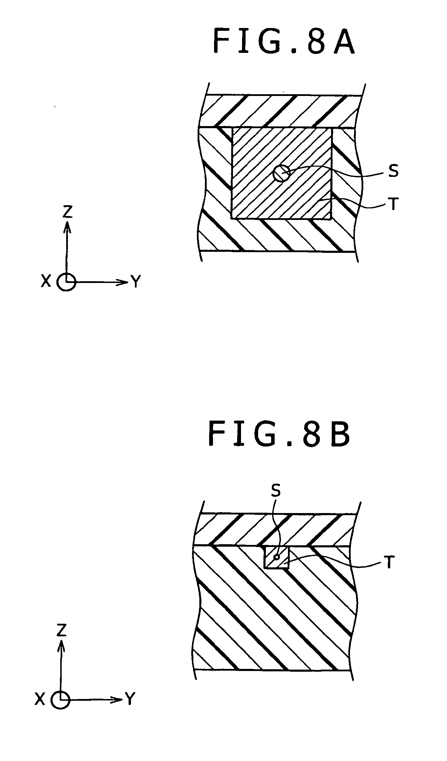

FIGS. 8A and 8B are schematic diagrams showing the sheath liquid laminar flow T and the sample liquid laminar flow S around the upstream side (FIG. 8A) and the downstream side (FIG. 8B) of the narrowing part 25. FIG. 8A is a schematic sectional view corresponding to the section along line M₁-M₁ in FIG. 6A, and FIG. 8B is a schematic sectional view corresponding to the section along line M₂-M₂ in FIG. 6A.

The feature that the narrowing part 25 is so formed that the microfluidic channel diameter gradually decreases in the Y-axis and Z-axis directions along the liquid sending direction can narrow the laminar flow widths of the sheath liquid laminar flow T and the sample liquid laminar flow S shown in FIG. 8A in the Y-axis and Z-axis directions as shown in FIG. 8B. In the present embodiment, simultaneously with the narrowing of the laminar flow widths of the sheath liquid laminar flow T and the sample liquid laminar flow S, both the laminar flows are biased toward the top surface of the micro-fluidic chip a (in the Z-axis positive direction in the diagram). By sending the liquids in such a way that the laminar flow widths of the sheath liquid laminar flow T and the sample liquid laminar flow S are narrowed in this manner, high detection sensitivity can be obtained in characteristic detection to be described next (details will be described later).

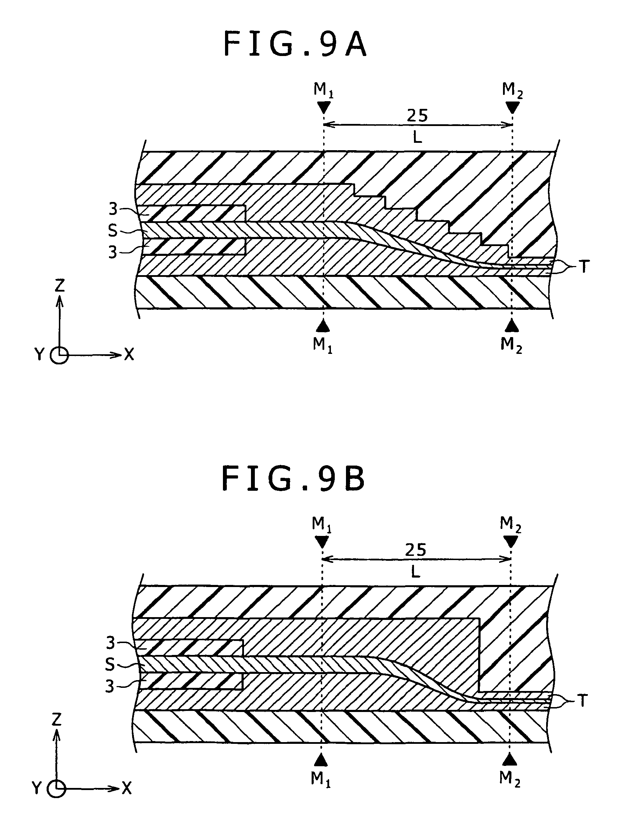

This narrowing of the laminar flow widths of the sheath liquid laminar flow T and the sample liquid laminar flow S can be achieved also by forming each of the microfluidic channel bottom surface and the top surface of the narrowing part 25 as an inclined surface. In addition, as shown in FIG. 9A, the microfluidic channel top surface (and/or the bottom surface) of the narrowing part 25 may be formed as a multi-stage surface having a shape of stairs from the upstream side toward the downstream side. In this case, also in top view, the narrowing part 25 is formed into a shape of stairs, which narrows the microfluidic channel in a stepwise manner. If the microfluidic channel diameter of the narrowing part 25 is decreased in the Y-axis and Z-axis directions in a stepwise manner along the liquid sending direction in this manner for the narrowing of the laminar flow widths, advantages in the shaping of the narrowing part 25 are achieved.

As described later, the shaping of the micro-fluidic chip a, the narrowing part 25, and so on can be carried out by wet etching or dry etching of a glass substrate layer, or nanoimprinting, injection molding, or cutting processing of a plastic substrate layer. The shaping of the narrowing part 25 is easier and particularly shaping by mechanical processing or optical molding can be carried out more easily when the narrowing part 25 has a shape of stairs than when it has an inclined surface.

For example, in the case of mechanical processing, a drill needs to be reciprocated many times in units of several micrometers for cutting in order to form a surface of the narrowing part 25 as an inclined surface. This process is very laborious. Furthermore, the wear of the drill easily progresses and a burr is often generated at the cut part. On the other hand, when the narrowing part 25 is formed into a shape of stairs with only several stages, cutting is easier and the wear of the drill is less and a burr is generated less readily. Also in the case of optical molding, when the narrowing part 25 is formed into a shape of stairs with only several stages, the number of times of the repetition of a CAD (Computer Aided Design) process and an optical molding process can be greatly decreased and thus the manufacturing time and the cost can be reduced. This point applies also to the case in which, as described above with FIG. 3, the bottom surface and so on of the region 1 is formed as a multi-stage surface with a shape of stairs in order to make magnetically-labeled cells be evenly absorbed by the surface facing the internal space of the region 1.

In the case of decreasing the microfluidic channel inner diameter of the narrowing part 25 in a stepwise manner in the liquid sending direction for narrowing, it is also possible to, as shown in FIG. 9B, decrease the microfluidic channel diameter to the inner diameter corresponding to the laminar flow widths resulting from the completion of the narrowing at one time.
Also in this case, the sheath liquid laminar flow T and the sample liquid laminar flow S can be narrowed in the Y-axis and Z-axis directions without the occurrence of a turbulent flow.

It may also be possible to form the sheath liquid laminar flow T and the sample liquid laminar flow S whose laminar flow widths are narrowed in advance, if the microfluidic channel 2 is formed as a sufficiently-thin microfluidic channel and the sample liquid is introduced into the sheath liquid laminar flow passing through this microfluidic channel 2 by using the microtube 3 whose diameter is small. However, this case possibly causes a problem that the microparticles contained in the sample liquid get stuck in the microtube 3 due to the small diameter of the microtube 3.

In the micro-fluidic chip a, the sample liquid laminar flow S and the sheath liquid laminar flow T are formed with use of the microtube 3 whose diameter is sufficiently larger than that of the microparticles contained in the sample liquid, and thereafter the laminar flow widths are narrowed by the narrowing part 25. This configuration can eliminate the problem of the clogging of the microtube 3.

The inner diameter of the microtube 3 can be accordingly set depending on the diameter of the microparticles contained in the sample liquid as the analysis subject. For example, the preferable inner diameter of the microtube 3 for analysis of cells is about 10 to 500 µm. Furthermore, the width and depth of the microfluidic channel 2 are accordingly set depending on the outer diameter of the microtube 3, which reflects the diameter of the microparticles as the analysis subject. For example, when the inner diameter of the microtube 3 is about 10 to 500 µm, it is preferable that each of the width and depth of the microfluidic channel 2 be about 100 to 2000 µm. The sectional shapes of the microtube 3 and the microfluidic channel 2 are not limited to a circular shape but may be any shape such as an ellipsoidal shape, a quadrangular shape, or a triangular shape.

The laminar flow widths of the sheath liquid laminar flow T and the sample liquid laminar flow S before the narrowing by the narrowing part 25 change depending on the width and depth of the microfluidic channel 2 and the diameter of the microtube 3. However, the laminar flow widths can be narrowed to any width by accordingly adjusting the microfluidic channel diameter of the narrowing part 25. For example, if the microfluidic channel length of the narrowing part 25 is defined as L and the inclination angle of the microfluidic channel bottom surface thereof is defined as θ_{Z} in FIG. 6A, the amount of narrowing of the laminar flow widths of the sheath liquid laminar flow T and the sample liquid laminar flow S in the narrowing part 25 is L·tanθ_{Z}. Therefore, any amount of narrowing can be set by accordingly adjusting the microfluidic channel length L and the inclination angle θ_{Z}. Furthermore, if the narrowing angles of the microfluidic channel side surfaces of the narrowing part 25 in the Y-axis direction are defined as θ_{Y1} and θ_{Y2} in FIG. 2 and these angles are equalized to the angle θ_{Z}, the sheath liquid laminar flow T and the sample liquid laminar flow S can be narrowed with isotropic width reduction as shown in FIGS. 8A and 8B.

### (4) Characteristic Detection by Detector

The sample liquid laminar flow S that is surrounded by the sheath liquid laminar flow T and has the laminar flow width narrowed by the narrowing part 25 is subjected to characteristic detection (optical characteristic detection, in this embodiment) by the detectors c₁ and c₂ (see FIG. 1). The sample liquid laminar flow S contains at least one of magnetically-unlabeled cells that are separately sent while a magnetic field is formed by the magnetic field generator b or magnetically-labeled cells that are sent thereafter while the magnetic field formation by the magnetic field generator b is stopped.

The detector c₁ focuses laser light on the magnetically-unlabeled cells or the magnetically-labeled cells contained in the sample liquid laminar flow S sent through the microfluidic channel 2 for irradiating the cells with the laser light. The detector c₂ detects light generated from the cells due to the laser light irradiation. The light detected by the detector c₂ may be scattered light or fluorescence, such as forward scattered light, side scattered light, Rayleigh scattered light, or Mie scattered light. The light is converted to an electrical signal and output to the overall controller f (see FIG. 1). Hereinafter, the position at which the characteristic detection by the detectors c₁ and c₂ is carried out in the microfluidic channel 2 will be referred to as "detection part."

In the microparticle analysis device A, the laminar flow widths of the sample liquid laminar flow S and the sheath liquid laminar flow T are narrowed by the narrowing part 25. Thus, the focus position of the laser light can be matched with the flow sending position of the cells at the detection part and the cells can be irradiated with the laser light with high accuracy. In particular, the narrowing part 25 can narrow the laminar flow width of the sample liquid laminar flow S not only in the horizontal direction of the micro-fluidic chip a (Y-axis direction in FIG. 2) but also in the vertical direction (Z-axis direction in FIGS. 6A and 6B). Thus, the focus position of the laser light in the depth direction of the microfluidic channel 2 can be exhaustively matched with the flow sending position of the cells. Accordingly, it is possible to irradiate the cells with the laser light with high accuracy and obtain high detection sensitivity.

Based on the input electrical signal, the overall controller f determines an optical characteristic of the cell by employing, as a parameter, the intensity of the scattered light or fluorescence, such as forward scattered light, side scattered light, Rayleigh scattered light, or Mie scattered light. The light employed as the parameter for the determination of the optical characteristic differs depending on the microparticle as the analysis subject. Specifically, forward scattered light is employed to determine the size of the microparticle, side scattered light is employed to determine the structure, and fluorescence is employed to determine whether or not a fluorescent substance as a label on the microparticle is present. The result of the determination of the optical characteristic by the overall controller f is output to the magnetic field generator b and the liquid sending controllers e₁ and e₂.

Although the detectors c₁ and c₂ are formed as an optical detection system in the present example, it is also possible to form them as an electrical detection system or a magnetic detection system. If the detectors c₁ and c₂ are formed as an electrical detection system or a magnetic detection system, an electrical characteristic or a magnetic characteristic is detected as a characteristic of the microparticle. In this case, microelectrodes are disposed as the detectors. Furthermore, e.g. any of the resistance, the capacitance, the inductance, the impedance, and the value of change in an electric field between the electrodes is measured. Alternatively, e.g. any of magnetization and a magnetic field change relating to the microparticles is measured. Two or more of these characteristics may be simultaneously measured. Also in the case of measuring an electrical or magnetic characteristic of microparticles, the measurement position of the microelectrodes can be exhaustively matched with the flow sending position of the microparticles and the characteristic can be detected with high sensitivity due to the effect of the narrowing part 25 similarly. The magnetic detectors can also determine whether or not a magnetic antibody attaches to microparticles sent in the sample flow via the internal space. Therefore, it is also possible to measure the amount of magnetically-labeled cells that should be held in the internal space originally but could not be separated and the purity and concentration of magnetically-labeled cells with respect to magnetically-unlabeled cells.

### (5) Concentration of Sample Liquid by Sheath Liquid Discharge Path

In FIG. 2, numerals 26 and 27 denote sheath liquid discharge paths arising from branching into trifurcate paths from the microfluidic channel 2 as the center of the branching. The position of the branching from the microfluidic channel 2 into the sheath liquid discharge paths 26 and 27 is set closer to the downstream side in the liquid sending direction than the detection part of the microfluidic channel 2. The sheath liquid discharge paths 26 and 27 function to separate only the sheath liquid laminar flow T from the sheath liquid laminar flow T and the sample liquid laminar flow S after the characteristic detection by the detectors c₁ and c₂ and send the concentrated sample liquid to the sample liquid outlet 21. The sheath liquid separated into the sheath liquid discharge paths 26 and 27 is discharged from sheath liquid outlets indicated by numerals 261 and 271 to the outside of the micro-fluidic chip a.

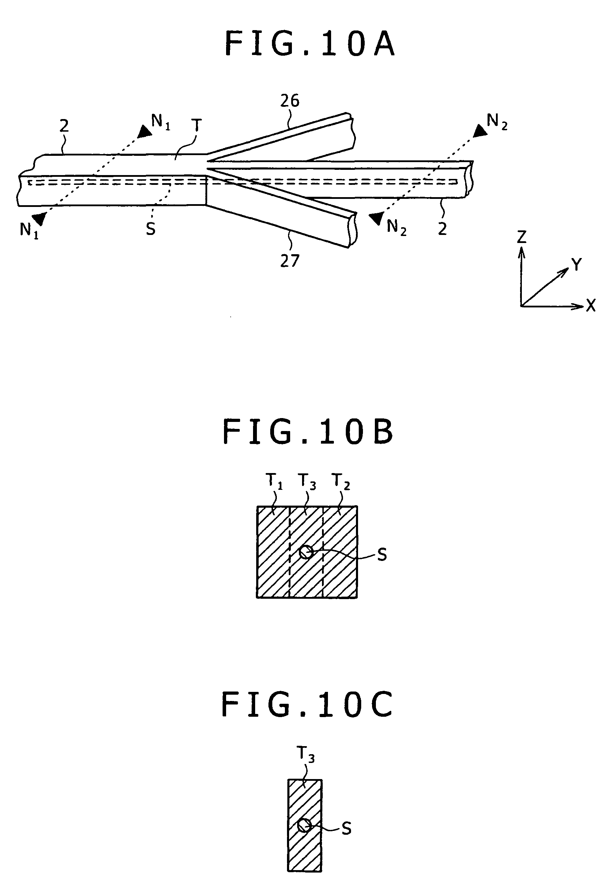

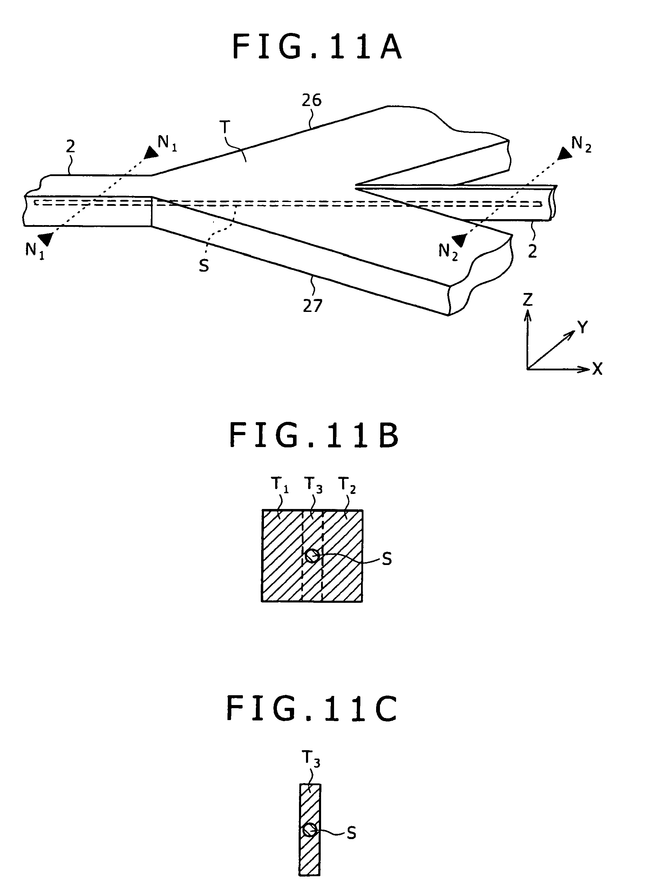

FIGS. 10A to 10C and 11A to 11C are diagrams schematically showing the sheath liquid laminar flow T and the sample liquid laminar flow S sent through the branch part for the sheath liquid discharge paths 26 and 27. In each of FIGS. 10A to 10C and 11A to 11C, each of 10A and 11A is a schematic perspective view, and 10B and 11B and 10C and 11C are sectional views. In each of FIGS. 10A to 10C and 11A to 11C, each of 10B and 11B corresponds to the section along line N₁-N₁ in each of 10A and 11A, and each of 10C and 11C corresponds to the section along line N₂-N₂ in each of 10A and 11A.

FIGS. 10A to 10C show the case in which the microfluidic channel 2 is branched into trifurcate paths at the branch part in such a way that the microfluidic channel widths of the sheath liquid discharge paths 26 and 27 in the Y-axis direction and the microfluidic channel width of the microfluidic channel 2 resulting from the branching are each 1/3 of that of the microfluidic channel 2 before the branching. In this case, of the sheath liquid laminar flow T on the upstream side of the branch part shown in FIG. 10B, partial portions T₁ and T₂ arising from division into three partial portions in the Y-axis direction at the branch part are separated into the sheath liquid discharge paths 26 and 27. Thus, the sheath liquid laminar flow T on the downstream side of the branch part is only a partial portion T₃ including the sample liquid laminar flow S as shown in FIG. 10C. Due to this separation of a part of the sheath liquid laminar flow T into the sheath liquid discharge paths, the sample liquid is concentrated by a factor of about three.

FIGS. 11A to 11C show the case in which the microfluidic channel widths of the sheath liquid discharge paths 26 and 27 in the Y-axis direction at the branch part are each 4/9 of that of the microfluidic channel 2 before the branching and the microfluidic channel width of the microfluidic channel 2 at the branch part is 1/9 of that of the microfluidic channel 2 before the branching. In this case, as shown in FIG. 11C, the sheath liquid laminar flow T on the downstream side of the branch part is only a partial portion T₃ that corresponds to a 1/9 portion arising from division into nine partial portions in the Y-axis direction and includes the sample liquid laminar flow S. Thus, the sample liquid is concentrated by a factor of about nine.

The rate of the separation of the sheath liquid laminar flow T into the sheath liquid discharge paths 26 and 27, i.e. the concentration rate of the sample liquid, can be set to any rate through adjustment of the ratio of the microfluidic channel widths of the sheath liquid discharge paths 26 and 27 to the microfluidic channel width of the microfluidic channel 2 at the branch part. The ratio of the microfluidic channel width between the sheath liquid discharge paths 26 and 27 and the microfluidic channel 2 at the branch part is so adjusted that the desired concentration rate is achieved, in consideration of the width and depth of the microfluidic channel 2, the inner diameter of the microtube 3, and so on at the time of laminar flow formation. In addition, the provision position of the microtube 3 in the microfluidic channel 2 also needs to be taken into consideration so that the partial portion T₃ including the sample liquid laminar flow S may be sent toward the downstream side of the microfluidic channel 2.

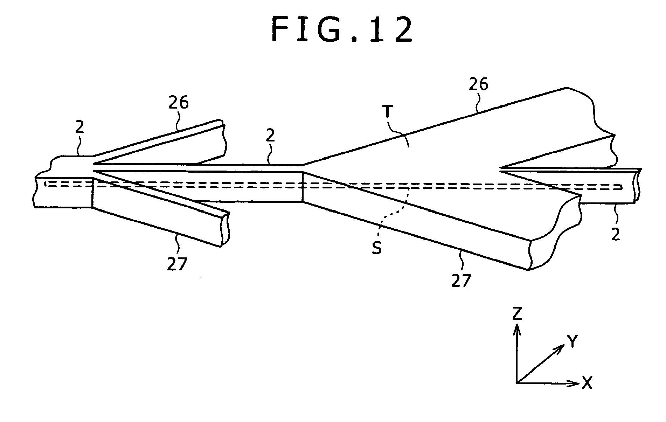

The number of branch parts of the microfluidic channel 2 is not limited to one but may be two or more. FIG. 12 shows the case in which two branch parts for the sheath liquid discharge paths are provided on the microfluidic channel 2. In the structure shown in the diagram, the branch part shown in FIG. 11A is connected to the microfluidic channel 2 on the downstream side of the branch part for the sheath liquid discharge paths 26 and 27 shown in FIG. 10A. In this example, the sample liquid can be concentrated by a factor of about three at the first branch part, and can be further concentrated by a factor of about nine at the next branch part. Accordingly, the sample liquid can be sent to the sample liquid outlet 21 after being concentrated by a factor of about 27.

### (6) Collection or Return of Sample Liquid by Liquid Sending Controller

The sample liquid that has passed through the branch part for the sheath liquid discharge paths and is discharged from the sample liquid outlet 21 to the outside of the micro-fluidic chip a is sent to either the collection tank d or the sample liquid inlet 11 selectively by the liquid sending controllers e₁ and e₂.

A signal is output from the overall controller f to the liquid sending controllers e₁ and e₂ (see FIG. 1). In response to this output, the liquid sending controllers e₁ and e₂ carry out control of switching of the sending destination of the sample liquid to either the collection tank d or the sample liquid inlet 11. The control by the liquid sending controllers e₁ and e₂ is carried out in linkage with the control by the magnetic field generator b, which forms a magnetic field or stops magnetic field formation in response to the output from the overall controller f. The details of the linked control of the liquid sending controllers e₁ and e₂ and the magnetic field generator b by the overall controller f will be described in the following chapter "microparticle analysis method."

### 3. Microparticle Analysis Method

A microparticle analysis method helpfull for the understanding of the present invention will be described below. The following description will deal with an example in which analysis and sorting of cells by the microparticle analysis device A are performed with use of a sample liquid containing magnetically-labeled cells as "magnetic microparticles" and magnetically-unlabeled cells as "non-magnetic microparticles." The cells as the sorting subject may be either the magnetically-unlabeled cells or the magnetically-labeled cells. The case of sorting the magnetically-unlabeled cells and the case of sorting the magnetically-labeled cells will be described below in turn.

### (1) Sorting of Magnetically-unlabeled cell (Negative Selection)

### (1-1) Cell Labeling

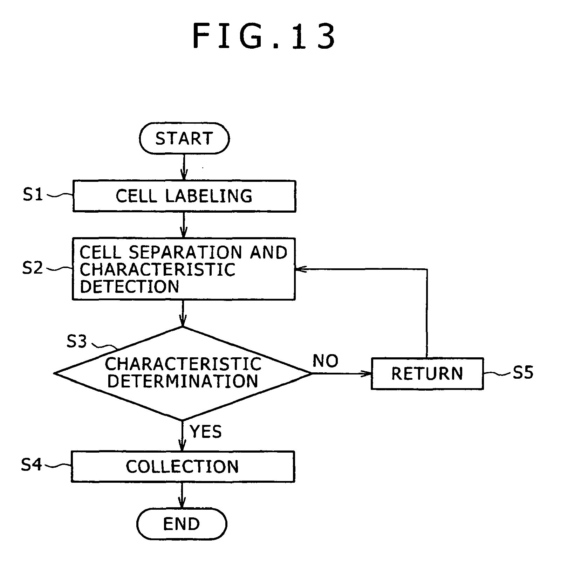

Initially, a method in which the magnetically-unlabeled cells are the sorting subject (hereinafter, referred to as "negative selection method") will be described below with reference to FIG. 13 and FIG. 1. In the negative selection method, of the cells contained in the sample liquid, the cells that are not the sorting subject (non-target cells) are magnetically labeled by using a magnetic bead antibody (see a step S1 in FIG. 13 and "Table 1"). On the other hand, the cells that are the sorting subject (target cells) are labeled with a substance that can be detected by the detectors c₁ and c₂. For example, if the detectors c₁ and c₂ are formed as an optical detection system, the target cells are fluorescent-labeled by using a fluorescent-labeled antibody. If the detectors c₁ and c₂ are an electrical or magnetic detector, the target cells are electrically or magnetically labeled.

**[Table 1]**

| <Negative Selection Method 1> |
|---|
| target cell: e.g. fluorescent-labeled |
| non-target cell: magnetically labeled |

### (1-2) Cell Separation and Characteristic Detection

The sample liquid containing the labeled cells is introduced from the sample liquid inlet 11 of the micro-fluidic chip a into the region 1 (see a step S2 in FIG. 13). A magnetic field by the magnetic field generator b is formed in the internal space of the region 1, and the target cells and the non-target cells contained in the sample liquid introduced into the region 1 are sent in the magnetic space generated by the magnetic field generator b via the turn-back parts 12 and 13 (see FIG. 1).

At this time, magnetic acting force works between the magnetically-labeled non-target cells and the magnetic field. Due to this magnetic acting force, the non-target cells are so held in the region 1 as to remain in the internal space of the region 1 against the liquid sending pressure of the sample liquid or be absorbed by the surface facing the internal space of the region 1. As a result, the cells contained in the sample liquid are separated into the non-target cells, which do not pass through the magnetic space generated by the magnetic field generator b, and the magnetically-unlabeled target cells, which pass through the magnetic space, and the sample liquid containing only the target cells is introduced from the end part of the region 1 via the microtube 3 into the microfluidic channel 2.

The sample liquid introduced into the microfluidic channel 2 is turned to a laminar flow surrounded by a sheath liquid laminar flow and its laminar flow width is narrowed by the narrowing part 25. Thereafter, the sample liquid laminar flow is subjected to characteristic detection by the detectors c₁ and c₂. The sample liquid laminar flow sent to the detection part contains the target cells, which are fluorescent-labeled for example. In addition to the target cells, a part of the non-target cells that are not separated but introduced into the microfluidic channel 2 due to failure in sufficient working of the magnetic acting force in the region 1 is also contained in the sample liquid laminar flow possibly. About these cells, the detectors c₁ and c₂ carry out characteristic detection based on e.g. fluorescence generated from the fluorescent substance labeled on the target cells. Furthermore, the detectors c₁ and c₂ convert the detection result to an electrical signal and output the signal to the overall controller f.

### (1-3) Characteristic Determination and Collection or Return

### (1-3-1) Characteristic Determination

The overall controller f carries out characteristic determination about the cells detected by the detectors c₁ and c₂ based on the input electrical signal (see a step S3 in FIG. 13). The characteristic determination is carried out by performing gating of the detected cells by use of the intensity of the fluorescence from the fluorescent substance, the scattered light intensity, or the like as a parameter.

### (1-3-2) Collection

If the cells as a predetermined ratio of the detected cells fall within the gating for the cells as the sorting subject and it is determined that the ratio of the target cells to all of the cells passing through the microfluidic channel 2 is equal to or higher than a predetermined value, the overall controller f outputs a determination result "Yes" to the liquid sending controllers e₁ and e₂. In response to this output, the liquid sending controllers e₁ and e₂ send the sample liquid discharged from the sample liquid outlet 21 to the collection tank d (see a step S4 in FIG. 13). Thus, the sample liquid containing the target cells at the predetermined ratio can be collected in the collection tank d. At this time, dilution of the sample liquid to be collected is prevented by the sheath liquid discharge paths 26 and 27 (see FIGS. 10A to 10C and so on).

### (1-3-3) Return

On the other hand, if the ratio of the cells that fall within the gating for the cells as the sorting subject to the cells detected by the detectors c₁ and c₂ is lower than the predetermined value and it is determined that a large number of non-target cells pass through the microfluidic channel 2, the overall controller f outputs a determination result "No" to the liquid sending controllers e₁ and e₂. In response to this output, the liquid sending controllers e₁ and e₂ introduce the sample liquid discharged from the sample liquid outlet 21 to the sample liquid inlet 11 and return it into the region 1 (see a step S5 in FIG. 13). For the returned sample liquid, the step S2 of "Separation and Characteristic Determination" is carried out again. Dilution of the sample liquid to be returned is prevented by the sheath liquid discharge paths 26 and 27 (see FIGS. 10A to 10C and so on).

The steps of the characteristic determination S3, the return S5, and the separation and characteristic detection S2 are repeated until the cells as a predetermined ratio of the detected cells fall within the gating for the cells as the sorting subject and it is determined that the ratio of the target cells to all of the cells passing through the microfluidic channel 2 is equal to or higher than the predetermined value. At the timing when the ratio of the target cells has become equal to or higher than the predetermined value, the sample liquid is introduced into the collection tank d, so that the sample liquid containing the target cells at the predetermined ratio is collected.

As above, the sample liquid containing the target cells at the predetermined ratio can be collected by magnetically labeling the non-target cells and carrying out the separation between the target cells and the non-target cells in the region 1 until the ratio of the target cells to all of the cells passing through the microfluidic channel 2 becomes equal to or higher than the predetermined value. In this method, the cells do not need to be sorted into the sorting microfluidic channels one by one by electrodes or the like differently from a method in a related-art microparticle sorting device. Thus, the analysis time is shorter and the target cells can be efficiently sorted. In addition, the target cells with high purity can be collected into the collection tank d by repeating the steps of the characteristic determination S3, the return S5, and the separation and characteristic determination S2 until the ratio of the target cells to all of the cells passing through the microfluidic channel 2 becomes the desired value.

It is also possible to, in this negative selection method, carry out the step S1 of the cell labeling with the combination shown in "Table 2."

**[Table 2]**

| <Negative Selection Method 2> |
|---|
| target cell: not labeled |
| non-target cell: magnetically labeled and e.g. fluorescent-labeled |

### (2) Sorting of Magnetically-labeled Cell (Positive Selection)

### (2-1) Cell Labeling

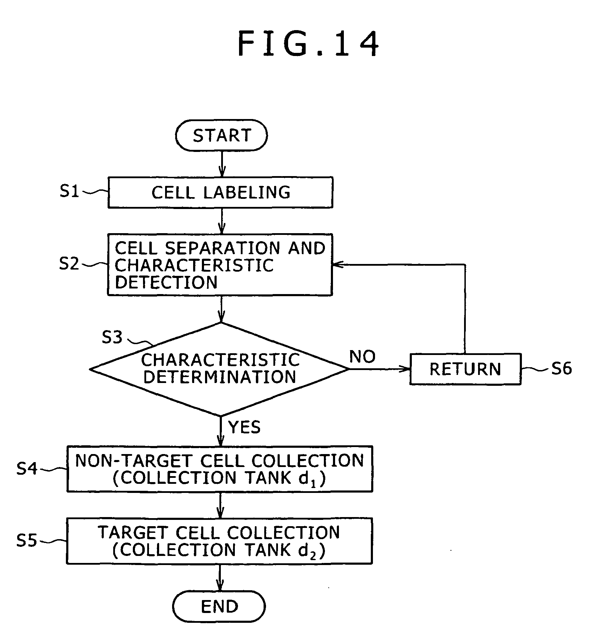

A method in which the magnetically-labeled cells are the sorting subject (hereinafter, referred to as "positive selection method") will be described below with reference to FIG. 14 and FIG. 1. In the positive selection method, for the target cells among the cells contained in the sample liquid, both magnetic labeling by use of a magnetic bead antibody and labeling with a substance that can be detected by the detectors c₁ and c₂ are carried out (see a step S1 in FIG. 14 and "Table 3"). On the other hand, the non-target cells are not labeled.

**[Table 3]**

| <Positive Selection Method 1> |
|---|
| target cell: magnetically labeled and e.g. fluorescent-labeled |
| non-target cell: not labeled |

### (2-2) Cell Separation and Characteristic Detection

The sample liquid containing the labeled cells is introduced from the sample liquid inlet 11 of the micro-fluidic chip a into the region 1 (see a step S2 in FIG. 14). A magnetic field by the magnetic field generator b is formed in the internal space of the region 1, and the target cells and the non-target cells contained in the sample liquid introduced into the region 1 are sent in the magnetic space generated by the magnetic field generator b via the turn-back parts 12 and 13.

At this time, magnetic acting force works between the magnetically-labeled target cells and the magnetic field. Due to this magnetic acting force, the target cells are so held in the region 1 as to remain in the internal space of the region 1 against the liquid sending pressure of the sample liquid or be absorbed by the surface facing the internal space of the region 1. As a result, the cells contained in the sample liquid are separated into the target cells, which do not pass through the magnetic space generated by the magnetic field generator b, and the magnetically-unlabeled non-target cells, which pass through the magnetic space, and the sample liquid containing only the non-target cells is introduced from the end part of the region 1 via the microtube 3 into the microfluidic channel 2.

The sample liquid introduced into the microfluidic channel 2 is turned to a laminar flow surrounded by a sheath liquid laminar flow and its laminar flow width is narrowed by the narrowing part 25. Thereafter, the sample liquid laminar flow is subjected to characteristic detection by the detectors c₁ and c₂. The sample liquid laminar flow sent to the detection part contains the non-target cells, which are not labeled. In addition to the non-target cells, a part of the target cells that are not separated but introduced into the microfluidic channel 2 due to failure in sufficient working of the magnetic acting force in the region 1 is also contained in the sample liquid laminar flow possibly. About these cells, the detectors c₁ and c₂ carry out characteristic detection based on e.g. fluorescence generated from the fluorescent substance labeled on the target cells. Furthermore, the detectors c₁ and c₂ convert the detection result to an electrical signal and output the signal to the overall controller f.

### (2-3) Characteristic Determination and Collection or Return

### (2-3-1) Characteristic Determination

The overall controller f carries out characteristic determination about the cells detected by the detectors c₁ and c₂ based on the input electrical signal (see a step S3 in FIG. 14). The characteristic determination is carried out by performing gating of the detected cells by use of the intensity of the fluorescence from the fluorescent substance or the scattered light intensity as a parameter.

### (2-3-2) Collection

If the ratio of the cells that fall within the gating for the cells as the sorting subject to the detected cells is lower than a predetermined value and it is determined that the number of target cells that are not separated but pass through the microfluidic channel 2 is sufficiently small, the overall controller f outputs a determination result "Yes" to the liquid sending controllers e₁ and e₂. In response to this output, the liquid sending controllers e₁ and e₂ send the sample liquid discharged from the sample liquid outlet 21 to a collection tank d₁ (see a step S4 in FIG. 14). Thus, the sample liquid containing the non-target cells is collected in the collection tank d₁. The non-target cells collected in the collection tank d₁ may be discarded as unnecessary cells.

Subsequently, the overall controller f outputs the determination result "Yes" to the magnetic field generator b to thereby stop the magnetic field formation. Due to this operation, the holding of the target cells in the region 1 is released, so that the target cells are sent to the microfluidic channel 2. Simultaneously, the overall controller f outputs the determination result also to the liquid sending controllers e₁ and e₂ to thereby introduce the sample liquid discharged from the sample liquid outlet 21 into a collection tank d₂ (see a step S5 in FIG. 14). Thus, the sample liquid containing the target cells at the predetermined ratio can be collected in the collection tank d₂. At this time, dilution of the sample liquid to be collected is prevented by the sheath liquid discharge paths 26 and 27 (see FIGS. 10A to 10C and so on).

The following operation may be carried out in the step S5 of the collection of the target cells. Specifically, after the magnetic field formation is stopped, the characteristic detection by the detectors c₁ and c₂ is carried out for the target cells sent to the microfluidic channel 2, and it is confirmed that the cells as a predetermined ratio of the detected cells fall within the gating for the target cells.

### (2-3-3) Return

On the other hand, if the ratio of the cells that fall within the gating for the cells as the sorting subject to the cells detected by the detectors c₁ and c₂ is equal to or higher than the predetermined value and it is determined that a large number of target cells pass through the microfluidic channel 2, the overall controller f outputs a determination result "No" to the liquid sending controllers e₁ and e₂. In response to this output, the liquid sending controllers e₁ and e₂ introduce the sample liquid discharged from the sample liquid outlet 21 to the sample liquid inlet 11 and return it into the region 1 (see a step S6 in FIG. 14). For the returned sample liquid, the step S2 of "Separation and Characteristic Determination" is carried out again. Dilution of the sample liquid to be returned is prevented by the sheath liquid discharge paths 26 and 27 (see FIGS. 10A to 10C and so on).

The steps of the characteristic determination S3, the return S6, and the separation and characteristic detection S2 are repeated until the ratio of the cells that fall within the gating for the cells as the sorting subject is lower than the predetermined value and it is determined that the number of target cells that are not separated but pass through the microfluidic channel 2 is sufficiently small. At the timing when the ratio of the target cells has become lower than the predetermined value, the sample liquid containing the non-target cells is introduced into the collection tank d₁ first. Subsequently, after the magnetic field formation by the magnetic field generator b is stopped, the sample liquid containing the target cells is introduced into the collection tank d₂, so that the sample liquid containing the target cells at the predetermined ratio is collected.

As above, the sample liquid containing the target cells at the predetermined ratio can be collected also by magnetically labeling the target cells and carrying out the separation between the target cells and the non-target cells in the region 1 until the ratio of the target cells to all of the cells passing through the microfluidic channel 2 becomes lower than the predetermined value.

It is also possible to, in this positive selection method, carry out the step S1 of the cell labeling with the combination shown in "Table 4."

**[Table 4]**

| <Positive Selection Method 2> |
|---|
| target cell: magnetically labeled |
| non-target cell: e.g. fluorescent-labeled |

### 4. Micro-fluidic Chip

The micro-fluidic chip a for microparticle analysis according to the embodiment of the present invention has a configuration including the region 1, the microfluidic channel 2, the microtube 3, and so on, which are described above for the microparticle analysis device A. The micro-fluidic chip a may include the magnetic field generator b for forming a magnetic field in the internal space of the region 1. In this case, the magnetic field generator b is provided by fabricating it as an electromagnetic in the substrate of the micro-fluidic chip a or stacking an electromagnetic on the substrate. Details of the respective components and operation of the micro-fluidic chip a are as described above, and therefore description thereof is omitted.

Glass or any of various kinds of plastic (PP, PC, COP, PDMS) can be used as the material of the micro-fluidic chip a. In the case of detecting an optical characteristic of microparticles by the detectors c₁ and c₂, the micro-fluidic chip a is formed by using a material that has optical transparency, low autofluorescence, small wavelength dispersion, and few optical errors. Furthermore, in this case, it is preferable that a so-called hard coat layer used for an optical disk is stacked on the surface of the micro-fluidic chip a. If dirt such as a fingerprint is attached to the surface of the micro-fluidic chip a, possibly the amount of light passing therethrough is decreased and the optical analysis accuracy is lowered. In particular, if dirt is attached to the surface of the detection part at which the optical characteristic detection by the detectors c₁ and c₂ is carried out in the microfluidic channel 2, accurate analysis possibly fails. By stacking a hard coat layer excellent in the transparency and the dirt repellency on the surface of the micro-fluidic chip a, the lowering of the analysis accuracy due to such dirt can be prevented.

The hard coat layer can be formed by using a generally-used hard coat agent. For example, it can be formed by using a UV-curing hard coat agent to which an agent for preventing fingerprint attachment, such as a fluorine-based or silicon-based dirt repellent additive agent, is added. Japanese Patent Laid-open No. 2003-157579 discloses, as the hard coat agent, (P) an active-energy-beam-curing composition including the following substances: (A) a polyfunctional compound having at least two polymerizable functional groups that can be polymerized by an active energy beam, (B) modified colloidal silica that has an average particle diameter of 1 to 200 nm and has the surface modified with a mercaptosilane compound in which an organic group having a mercapto group and a hydrolyzable group or a hydroxyl group are coupled to a silicon atom, and (C) a photopolymerization initiator.

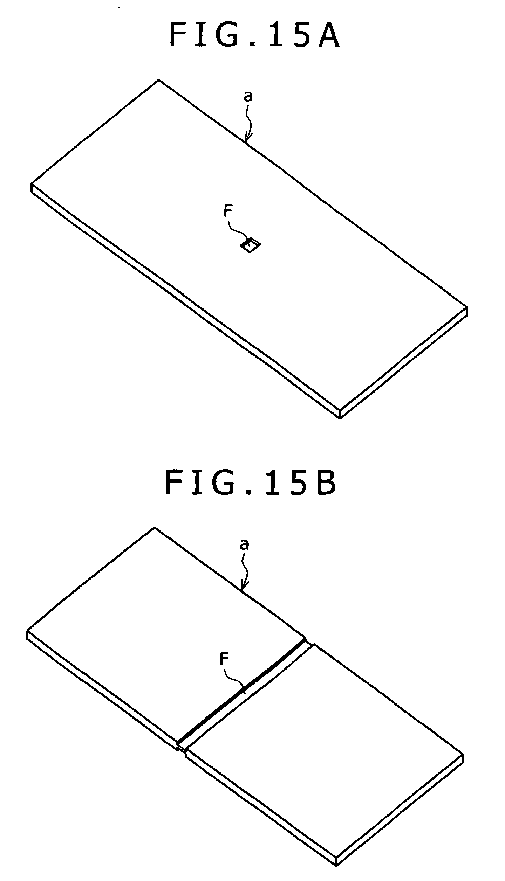

For preventing dirt attachment to the surface of the detection part in the microfluidic channel 2, it is also effective to, as shown in FIGS. 15A and 15B, recess the surface of the detection part (see symbol F in the diagram) relative to the surface of the other part of the micro-fluidic chip a to thereby prevent contact of a fingertip and dirt to the surface of the detection part. FIG. 15A shows the case in which a dimple is formed at the part corresponding to the detection part on the surface of the micro-fluidic chip a. FIG. 15B shows the case in which a groove including the part corresponding to the detection part is formed on the surface of the micro-fluidic chip a.

As the microtube 3, a tube composed of a metal, glass, ceramics, or any of various kinds of plastic (PP, PC, COP, PDMS) can be employed. As for a silica tube, tubes each having an inner diameter of several tens to several hundreds of micrometers are commercially available. Therefore, the silica tube can be favorably employed as the microtube 3 because a tube having a suitable diameter can be accordingly utilized. The silica tube has high heat resistance, and therefore thermal deformation hardly occurs even in thermocompression bonding of the substrate layer.

The shaping of the region 1, the microfluidic channel 2, and so on provided on the micro-fluidic chip a can be carried out by wet etching or dry etching of a glass substrate layer, or nanoimprinting, injection molding, or cutting processing of a plastic substrate layer. The microtube 3 is disposed on the substrate layer in which the region 1 and so on is shaped in such a manner as to be fitted into a groove formed to allow communication between the end part of the region 1 and the microfluidic channel 2. After the microtube is disposed, the substrate layer in which the region 1 and so on is shaped is covered and sealed by a substrate layer composed of the same material or a different material, so that the micro-fluidic chip a is formed.

The bonding of the substrate layers can be carried out e.g. by any of the following publicly-known methods: heat fusion, an adhesive, anodic bonding, bonding by use of an adhesive sheet, plasma-activated bonding, and ultrasonic bonding. The groove into which the microtube 3 is fitted is sealed by an adhesive for fixing the microtube 3 to the substrate layer. Due to the sealing of the groove, the communication between the region 1 and the microfluidic channel 2 is achieved only via the microtube 3. This allows the sample liquid sent to the end part of the region 1 to be introduced into the microfluidic channel 2 by the microtube 3.

The present application contains subject matter related to that disclosed in Japanese Priority Patent Application JP 2009-001471 filed in the Japan Patent Office on January 7, 2009.

It should be understood by those skilled in the art that various modifications, combinations, sub-combinations and alternations may occur depending on design requirements and other factors insofar as they are within the scope of the appended claims.

## Claims

1. A micro-fluidic chip for microparticle analysis, comprising:
a region configured to have an internal space adapted to form In the internal space a magnetic field by a magnetic field generating **means,**
an inlet configured to introduce a sample liquid containing a magnetic microparticle and a non-magnetic microparticle into the region; and
a microfluidic channel configured to communicate with the region on a downstream side in a liquid sending direction, and configured for detection of a microparticle in the microfluidic channel by an optical, electrical, or magnetic detecting means being carried out, **characterized by**
a microtube configured to be connected to the region and introduce a sample liquid sent from the region into the microfluidic channel to form a sample liquid laminar flow in a sheath liquid laminar flow passing through the microfluidic channel; wherein
the microfluidic channel has a narrowing part at a position that is closer to the downstream side in the liquid sending direction than a position of introduction of a sample liquid by the microtube and is closer to an upstream side in the liquid sending direction than a detection part at which detection of a characteristic of a microparticle by the detecting means is carried out, and a microfluidic channel inner diameter of the narrowing part is decreased gradually or in a stepwise manner in the liquid sending direction;
the microfluidic channel has two sheath liquid discharge paths arising from branching into trifurcate paths from the microfluidic channel as a center of the branching at a branch part closer to the downstream side in the liquid sending direction than the detection part; and
wherein a magnetic tube having a hollow shape is provided in the internal space of the region in such a way that a longitudinal direction of the magnetic tube corresponds with the liquid sending direction.

2. The micro-fluidic chip for microparticle analysis according to claim 1. wherein
a space inner diameter of the internal space of the region along a direction of a magnetic field formed by the magnetic field generating means is increased gradually or in a stepwise manner in the liquid sending direction.

3. The micro-fluidic chip for microparticle analysis according to claim 1, wherein
the region is formed as a turn-back microfluidic channel made in a magnetic space generated by the magnetic field generating means.

4. The micro-fluidic chip for microparticle analysis according to claim 1, wherein
small projections and recesses are processed on a region surface facing the internal space of the region or the region surface is coated with a magnetic material.

5. A microparticle analysis device comprising:
the micro-fluidic chip for microparticle analysis according to claim 1;
magnetic field generating means for forming a magnetic field in the internal space of the region of the micro-fluidic chip; and
detecting means for detecting an optical, electrical, or magnetic characteristic of a microparticle passing through the microfluidic channel.

6. The microparticle analysis device according to claim 5, further comprising
liquid sending controlling means for returning a sample liquid sent to a position closer to the downstream side in the liquid sending direction than the branch part of the microfluidic channel into the region or introducing the sample liquid into a collection tank.

7. A microparticle analysis method comprising the steps of:
introducing a sample liquid containing a magnetic microparticle and a non-magnetic microparticle into a region on a micro-fluidic chip, having an internal space in which a magnetic field is formed by magnetic field generating means;
holding the magnetic microparticle that does not pass through a magnetic space generated by the magnetic field generating means in the region and simultaneously sending the non-magnetic microparticle that passes through the magnetic space generated by the magnetic field generating means to a microfluidic channel connected to the region on a downstream side in a liquid sending direction; and
analyzing a characteristic of the microparticle in the microfluidic channel by an optical, electrical, or magnetic detecting means or
collecting the non-magnetic microparticle: **characterized by**
holding the magnetic microparticle on the hollow surface and the outside surface of a magnetic tube in the internal space having a hollow shape being provided in such a way that a longitudinal direction of the magnetic tube corresponds with the liquid sending direction;
sending the non-magnetic microparticle from the region to the microfluidic channel through a microtube that is connected to the region by introducing the sample liquid sent from the region into the microfluidic channel;
forming a sample liquid laminar flow in a sheath liquid laminar flow passing through the microfluidic channel;
branching the flow from the microfluidic channel into trifurcate paths including two sheath liquid discharge paths wherein the center of the branching at a branch part is closer to the downstream side in the liquid sending direction than the detection means;
returning a sample liquid sent to a position closer to the downstream side in the liquid sending direction than the branch part of the microfluidic channel into the region or introducing the sample liquid into a collection tank by a liquid sending controller; wherein
the microfluidic channel has a narrowing part at a position that is closer to the downstream side in the liquid sending direction than the position of introduction of the sample liquid by the microtube and is closer to the upstream side in the liquid sending direction than the detection means and a microfluidic channel inner diameter of the narrowing part is decreased gradually or in a stepwise manner in the liquid sending direction.

8. The microparticle analysis method according to claim 7, wherein the space inner diameter of the internal space of the region along the direction of a magnetic field formed by the magnetic field generator is increased gradually or in a stepwise manner in the liquid sending direction.

## Patentansprüche

1. Mikrofluidischer Chip zur Mikropartikel-Analyse, umfassend:
einen Bereich, ausgebildet, einen Innenraum aufzuweisen, geeignet, ein Magnetfeld in dem Innenraum durch eine ein Magnetfeld erzeugenden Einrichtung zu bilden,
einen Einlass, ausgebildet, eine Probenflüssigkeit, die einen magnetischen Mikropartikel und einen nicht-magnetischen Mikropartikel aufweist, in den Bereich einzuleiten; und
einen mikrofluidischen Kanal, ausgebildet, mit dem Bereich auf einer stromabwärtigen Seite in einer Flüssigkeitstransportrichtung zu kommunizieren, und ausgebildet zum Detektieren eines Mikropartikels in dem mikrofluidischen Kanal durch eine optische, elektrische oder magnetische Detektionseinrichtung, das durchgeführt wird, **gekennzeichnet durch**
ein Mikrorohr, ausgebildet, mit dem Bereich verbunden zu sein und eine Probenflüssigkeit, aus der Region in den mikrofluidischen Kanal geschickt, einzuleiten, um einen laminaren Probenflüssigkeitsstrom in einem laminaren Hüllflüssigkeitsstrom, **durch** den mikrofluidischen Kanal hindurchtretend, zu bilden; wobei
der mikrofluidische Kanal eine Verengungstelle an einer Position aufweist, die näher an der stromabwärtigen Seite in der Flüssigkeitstransportrichtung ist als eine Position einer Einleitung einer Probenflüssigkeit **durch** das Mikrorohr und näher an einer stromaufwärtigen Seite in der Flüssigkeitstransportrichtung ist als ein Detektionsteil, in dem eine Detektion einer Eigenschaft eines Mikropartikels **durch** die Detektionseinrichtung durchgeführt wird, und sich ein Innendurchmesser der Verengungsstelle des mikrofluidischen Kanals allmählich oder stufenweise in der Flüssigkeitstransportrichtung verringert;
der mikrofluidische Kanal zwei Ausleitwege für die Hüllflüssigkeit aufweist, entstehend **durch** Verzweigen in drei Pfade von dem mikrofluidischen Kanal als Zentrum der Verzweigung an einem Zweigteil näher an der stromabwärtigen Seite der Flüssigkeitstransportrichtung als der Detektionsteil; und
wobei ein magnetisches Rohr mit einer hohlen Form in dem Innenraum des Bereichs in einer Weise vorgesehen ist, dass eine Längsrichtung des magnetischen Rohrs mit der Flüssigkeitstransportrichtung korrespondiert.

2. Der mikrofluidische Chip für die Mikropartikel-Analyse nach Anspruch 1 , wobei
sich ein Raum-Innendurchmesser des Innenraums des Bereichs entlang einer Richtung eines Magnetfeldes, gebildet durch die ein Magnetfeld erzeugende Einrichtung, allmählich oder stufenweise in der Flüssigkeitstransportrichtung vergrößert.

3. Der mikrofluidische Chip für die Mikropartikel-Analyse nach Anspruch 1, wobei
der Bereich als Umkehr-mikrofluidischer Kanal, ausgebildet in einem magnetischen Raum, erzeugt durch die ein Magnetfeld erzeugende Einrichtung, gebildet wird.

4. Der mikrofluidische Chip für die Mikropartikel-Analyse nach Anspruch 1, wobei
kleine Vorsprünge und Vertiefungen auf einer Bereichsoberfläche, dem Innenraum des Bereichs zugewandt, gebildet werden oder die Bereichsoberfläche mit einem magnetischen Material beschichtet ist.

5. Mikropartikel-Analyseeinrichtung mit:
dem mikrofluidische Chip für die Mikropartikel-Analyse nach Anspruch 1;
einer ein Magnetfeld erzeugenden Einrichtung zum Erzeugen eines Magnetfelds in dem Innenraum des Bereichs des mikrofluidische Chips; und
einer Detektionseinrichtung zum Detektieren einer optischen, elektrischen oder magnetischen Eigenschaft eines Mikropartikels, der durch den mikrofluidischen Kanal hindurchtritt.

6. Die Mikropartikel-Analyseeinrichtung nach Anspruch 5, ferner aufweisend:
einen Flüssigkeitstransportsteuereinrichtung für einen Rücktransport einer Probenflüssigkeit, an eine Position näher der stromabwärtigen Seite in der Flüssigkeitstransportrichtung als dem Verzweigungsteil des mikrofluidischen Kanals transportiert, oder zum Einbringen der Probenflüssigkeit in einen Sammeltank.

7. Mikropartikel-Analyse-Verfahren, die Schritte aufweisend:
Einbringen einer einen magnetische Mikropartikel und einen nicht-magnetischen Mikropartikel enthaltenden Probenflüssigkeit in einen Bereich auf einem mikrofluidischen Chip mit einem Innenraum, in dem ein Magnetfeld durch eine ein Magnetfeld erzeugende Einrichtung gebildet wird;
Halten des magnetischen Mikropartikels, der durch einen magnetischen Raum, der durch die das Magnetfeld erzeugende Einrichtung gebildet wird, nicht hindurchtritt, in dem Bereich und gleichzeitiges Transportieren des nicht-magnetischen Mikropartikels, der durch einen magnetischen Raum, der durch die das Magnetfeld erzeugende Einrichtung gebildet wird, hindurchtritt, in einen mikrofluidischen Kanal, mit dem Bereich auf einer stromabwärtigen Seite in einer Flüssigkeitstransportrichtung verbunden; und
Analysieren einer Eigenschaft des Mikropartikels in dem mikrofluidischen Kanal durch eine optische, elektrische oder magnetische Detektionseinrichtung oder
Einsammeln des nicht-magnetischen Mikropartikels; **gekennzeichnet durch**
Halten des magnetischen Mikropartikels auf der Hohlkörperoberfläche und der äußeren Oberfläche einer magnetischen Röhre in dem Innenraum mit einer hohlen Form, derart vorgesehen, dass eine Längsrichtung der magnetischen Röhre mit der Flüssigkeitstransportrichtung korrespondiert;
Transportieren des nicht-magnetischen Mikropartikels aus dem Bereich in den mikrofluidischen Kanal **durch** eine Mikroröhre, die mit dem Bereich verbunden ist, **durch** Einleiten der Probenflüssigkeit, geschickt von dem Bereich in den mikrofluidischen Kanal;
Ausbilden eines laminaren Probenflüssigkeitsstroms in einem laminaren Hüllflüssigkeitsstrom, **durch** den mikrofluidischen Kanal hindurchtretend;
Aufteilen des Stroms von dem mikrofluidischen Kanal in drei Pfade mit zwei Hüllflüssigkeitsableitpfaden, wobei das Zentrum der Verzweigung an einem Zweigteil näher an der stromabwärtigen Seite der Flüssigkeitstransportrichtung als die Detektionseinrichtung liegt;
Zurückführen der Probenflüssigkeit, transportiert zu einer Position näher zu der stromabwärtigen Seite der Flüssigkeitstransportrichtung als der Verzweigungsteil des mikrofluidischen Kanals, in den Bereich oder Einleiten der Probenflüssigkeit in einen Sammeltank **durch** eine Flüssigkeitstransportsteuerung; wobei
der mikrofluidische Kanal eine Verengung an einer Position aufweist, die näher an der stromabwärtigen Seite in der Flüssigkeitstransportrichtung ist als eine Position einer Einleitung einer Probenflüssigkeit **durch** das Mikrorohr und näher an einer stromaufwärtigen Seite in der Flüssigkeitstransportrichtung ist als die Detektionseinrichtung und sich ein Innendurchmesser der Verengungsstelle des mikrofluidischen Kanals allmählich oder stufenweise in der Flüssigkeitstransportrichtung verringert;

8. Das Mikropartikel-Analyse-Verfahren nach Anspruch 7, wobei sich ein Raum-Innendurchmesser des Innenraums des Bereichs entlang einer Richtung eines Magnetfeldes, gebildet durch die ein Magnetfeld erzeugende Einrichtung, allmählich oder stufenweise in der Flüssigkeitstransportrichtung vergrößert.

## Revendications

1. Puce microfluidique pour l'analyse de microparticules, comprenant :
une région configurée pour comporter un espace interne conçu pour former, dans l'espace interne, un champ magnétique par des moyens de génération de champ magnétique ;
une entrée configurée pour introduire un échantillon liquide contenant une microparticule magnétique et une microparticule non magnétique dans la région ; et
un canal microfluidique configuré pour communiquer avec la région d'un côté aval dans une direction d'envoi de liquide, et configuré pour que la détection d'une microparticule dans le canal microfluidique par des moyens de détection optiques, électriques ou magnétiques soit effectuée, **caractérisée par**
un microtube configuré pour être relié à la région et pour introduire un échantillon liquide envoyé de la région dans le canal microfluidique pour former un écoulement laminaire d'échantillon liquide dans un écoulement laminaire de liquide de gaine passant à travers le canal microfluidique ; dans laquelle
le canal microfluidique comporte une partie qui rétrécit à une position qui est plus proche du côté aval dans la direction d'envoi de liquide qu'une position d'introduction d'un échantillon liquide par le microtube et qui est plus proche d'un côté amont dans la direction d'envoi de liquide qu'une partie de détection au niveau de laquelle la détection d'une caractéristique d'une microparticule par les moyens de détection est effectuée, et un diamètre interne de canal microfluidique de la partie qui rétrécit est diminué graduellement ou par pas dans la direction d'envoi de liquide ;
le canal microfluidique comporte deux trajets de décharge de liquide de gaine provenant d'une division en trajets trifurqués du canal microfluidique en tant que centre de la division au niveau d'une partie de division plus proche du côté aval dans la direction d'envoi de liquide que la partie de détection ; et
dans laquelle un tube magnétique ayant une forme creuse est prévu dans l'espace interne de la région d'une manière telle qu'une direction longitudinale du tube magnétique correspond à la direction d'envoi de liquide.

2. Puce microfluidique pour l'analyse de microparticules selon la revendication 1, dans laquelle
un diamètre interne d'espace de l'espace interne de la région le long d'une direction d'un champ magnétique formé par les moyens de génération de champ magnétique est augmenté graduellement ou par pas dans la direction d'envoi de liquide.

3. Puce microfluidique pour l'analyse de microparticules selon la revendication 1, dans laquelle
la région est formée en tant que canal microfluidique de demi-tour réalisé dans un espace magnétique généré par les moyens de génération de champ magnétique.

4. Puce microfluidique pour l'analyse de microparticules selon la revendication 1, dans laquelle
de petites protubérances et de petits évidements sont traités sur une surface de la région faisant face à l'espace interne de la région ou la surface de la région est revêtue d'un matériau magnétique.

5. Dispositif d'analyse de microparticules comprenant :
la puce microfluidique pour l'analyse de microparticules selon la revendication 1 ;
des moyens de génération de champ magnétique pour former un champ magnétique dans l'espace interne de la région de la puce microfluidique ; et
des moyens de détection pour détecter une caractéristique optique, électrique ou magnétique d'une microparticule passant à travers le canal microfluidique.

6. Dispositif d'analyse de microparticules selon la revendication 5, comprenant en outre :
des moyens de commande d'envoi de liquide pour renvoyer un échantillon liquide envoyé vers une position plus proche du côté aval dans la direction d'envoi de liquide que la partie de division du canal microfluidique dans la région ou pour introduire l'échantillon liquide dans un réservoir de collecte.

7. Procédé d'analyse de microparticules comprenant les étapes consistant à :
introduire un échantillon liquide contenant une microparticule magnétique et une microparticule non magnétique dans une région sur une puce microfluidique, comportant un espace interne dans lequel un champ magnétique est formé par des moyens de génération de champ magnétique ;
maintenir la microparticule magnétique qui ne passe pas à travers un espace magnétique généré par les moyens de génération de champ magnétique dans la région et envoyer simultanément la microparticule non magnétique qui passe à travers l'espace magnétique généré par les moyens de génération de champ magnétique vers un canal microfluidique relié à la région d'un côté aval dans une direction d'envoi de liquide ; et
analyser une caractéristique de la microparticule dans le canal microfluidique par des moyens de détection optiques, électriques ou magnétiques, ou
collecter la microparticule non magnétique ; **caractérisé par**
le maintien de la microparticule magnétique sur la surface creuse et la surface extérieure d'un tube magnétique dans l'espace interne ayant une forme creuse prévu d'une manière telle qu'une direction longitudinale du tube magnétique correspond avec la direction d'envoi de liquide ;
l'envoi de la microparticule non magnétique de la région vers le canal microfluidique à travers un microtube qui est relié à la région en introduisant l'échantillon liquide envoyé de la région dans le canal microfluidique ;
la formation d'un écoulement laminaire d'échantillon liquide dans un écoulement laminaire de liquide de gaine passant à travers le canal microfluidique ;
la division de l'écoulement provenant du canal microfluidique en trajets trifurqués comprenant deux trajets de décharge de liquide de gaine, dans lequel le centre de la division au niveau d'une partie de division est plus proche du côté aval dans la direction d'envoi de liquide que les moyens de détection ;
le renvoi d'un échantillon liquide envoyé vers une position plus proche du côté aval dans la direction d'envoi de liquide que la partie de division du canal microfluidique dans la région ou l'introduction de l'échantillon liquide dans un réservoir de collecte par un contrôleur d'envoi de liquide ; dans lequel
le canal microfluidique comporte une partie qui rétrécit à une position qui est plus proche du côté aval dans la direction d'envoi de liquide que la position d'introduction de l'échantillon liquide par le microtube et qui est plus proche du côté amont dans la direction d'envoi de liquide que les moyens de détection, et un diamètre interne de canal microfluidique de la partie qui rétrécit est diminué graduellement ou par pas dans la direction d'envoi de liquide.

8. Procédé d'analyse de microparticules selon la revendication 7, dans lequel le diamètre interne d'espace de l'espace interne de la région le long de la direction d'un champ magnétique formé par le générateur de champ magnétique est augmenté graduellement ou par pas dans la direction d'envoi de liquide.
